(19) Europäisches Patentamt
European Patent Office
Office européen des brevets

(11) EP 1 603 907 B1

(12) **EUROPÄISCHE PATENTSCHRIFT**

(45) Veröffentlichungstag und Bekanntmachung des Hinweises auf die Patenterteilung:
**30.08.2006 Patentblatt 2006/35**

(21) Anmeldenummer: **04717586.4**

(22) Anmeldetag: **05.03.2004**

(51) Int Cl.:
***C07D 471/04*** (2006.01) ***A61K 31/519*** (2006.01)
***A61P 29/00*** (2006.01) *C07D 471/04* (2006.01)
*C07D 239/00* (2006.01) *C07D 221/00* (2006.01)

(86) Internationale Anmeldenummer:
**PCT/EP2004/002279**

(87) Internationale Veröffentlichungsnummer:
**WO 2004/078755 (16.09.2004 Gazette 2004/38)**

(54) **SUBSTITUIERTE PYRIDO(1,2-A)PYRIMIDINE UND IHRE VERWENDUNG ALS NOS-INHIBITOREN**

SUBSTITUTED PYRIDO(1,2-A)PYRIMIDINES AND THEIR USE AS NOS INHIBITORS

COMPOSES DE PYRIDO(1,2-A)PYRIMIDINE SUBSTITUES ET LEUR UTILISATION COMME INHIBITEURS DE NOS

(84) Benannte Vertragsstaaten:
**AT BE BG CH CY CZ DE DK EE ES FI FR GB GR HU IE IT LI LU MC NL PL PT RO SE SI SK TR**
Benannte Erstreckungsstaaten:
**LT LV**

(30) Priorität: **06.03.2003 DE 10310106**

(43) Veröffentlichungstag der Anmeldung:
**14.12.2005 Patentblatt 2005/50**

(73) Patentinhaber: **Grünenthal GmbH**
**52078 Aachen (DE)**

(72) Erfinder:
- **SUNDERMANN, Bernd**
  **52074 Aachen (DE)**
- **HENNIES, Hagen-Heinrich**
  **52152 Simmerath (DE)**
- **BUSCHMANN, Helmut**
  **E-08950 Esplugues de Llobregat (ES)**
- **BLUHM, Ullvi**
  **24119 Kronshagen (DE)**
- **CLEMENT, Bernd**
  **24106 Kiel (DE)**
- **HEBER, Dieter**
  **24113 Molfsee (DE)**
- **WOLSCHENDORF, Ulrich**
  **24582 Bordesholm (DE)**

(74) Vertreter: **Kutzenberger, Helga et al**
**Kutzenberger & Wolff,**
**Theodor-Heuss-Ring 23**
**50668 Köln (DE)**

(56) Entgegenhaltungen:
**WO-A-02/080911**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft substituierte Pyrido[1,2-a]pyrimidin-Verbindungen, Verfahren zu deren Herstellung, Arzneimittel enthaltend diese Verbindungen sowie deren Verwendung zur Herstellung von Arzneimitteln.

Stickstoffmonoxid (NO) reguliert zahlreiche physiologische Prozesse, unter anderem die Neurotransmission, die Relaxation und Proliferation von glatter Muskulatur, die Adhäsion und Aggregation von Thrombozyten sowie die Gewebeverletzung und Entzündung. Aufgrund der Vielzahl von Signalfunktionen wird Stickstoffmonoxid mit einer Reihe von Krankheiten in Verbindung gebracht, beispielsweise in L. J. Ignarro, Angew. Chem. (1999), 111, Seiten 2002-2013 und in F. Murad, Angew. Chem. Int. Ed. (1999), 111, Seiten 1976-1989. Eine wichtige Rolle bei der therapeutischen Beeinflussung dieser Krankheiten spielt dabei das für die physiologische Bildung von Stickstoffmonoxid verantwortliche Enzym, die Stickstoffmonoxid-Synthase (NO-Synthase). Bislang wurden drei verschiedene Isoformen der NO-Synthase identifiziert, nämlich die beiden konstitutiven Formen nNO-Synthase und eNO-Synthase sowie die induzierbare Form iNO-Synthase (A. J. Hobbs, A. Higgs, S. Moncada, Annu. Rev. Pharmacol. Toxicol. (1999), 39, Seiten 191-220; I. C. Green, P.-E. Chabrier, DDT (1999), 4, Seiten 47-49; P.-E. Chabrier et al., Cell. Mol. Life Sci. (1999), 55, Seiten 1029-1035).

Die Hemmung der NO-Synthase eröffnet neue Therapieansätze für verschiedene Krankheiten, die mit Stickstoffmonoxid in Zusammenhang stehen (A. J. Hobbs et al., Annu. Rev. Pharmacol. Toxicol. (1999), 39, Seiten 191-220; I. C. Green, P.-E. Chabrier, DDT (1999), 4, Seiten 47-49; P.-E. Chabrier et al., Cell. Mol. Life Sci. (1999), 55, Seiten 1029-1035), wie beispielsweise Migräne (L. L. Thomsen, J. Olesen, Clinical Neuroscience (1998), 5, Seiten 28-33; L. H. Lassen et al., The Lancet (1997), 349, 401-402), septischer Schock, neurodegenerative Erkrankungen wie Multiple Sklerose, Morbus Parkinson, Morbus Alzheimer oder Morbus Huntington, Entzündungen, Entzündungsschmerz, cerebrale Ischämie, Diabetes, Meningitis und Arteriosklerose. Darüber hinaus kann die Inhibierung der NO-Synthase einen Effekt auf die Wundheilung, auf Tumoren und auf die Angiogenese haben sowie eine unspezifische Immunität gegen Mikroorganismen bewirken (A. J. Hobbs et al., Annu. Rev. Pharmacol. Toxicol. (1999), 39, Seiten 191-220).

Bislang bekannte Wirkstoffe, die die NO-Synthase hemmen, sind neben L-NMMA und L-NAME -d.h. Analoga des L-Arginins, aus dem in-vivo unter Beteiligung von NO-Synthase Stickstoffmonoxid und Citrullin gebildet werden,- u.a. S-Methyl-L-citrullin, Aminoguanidin, S-Methylisoharnstoff, 7-Nitroindazol, 2-Mercaptoethylguanidin (A. J. Hobbs et al., Annu. Rev. Pharmacol. Toxicol. (1999), 39, Seiten 191-220), und Imidazo[1,2-a]-pyridinderivate (WO-A-02/080911).

Eine Aufgabe der vorliegenden Erfindung bestand daher darin, neue Verbindungen zur Verfügung zur stellen, die sich insbesondere als pharmazeutische Wirkstoffe in Arzneimitteln eignen, vorzugsweise in Arzneimitteln zur Inhibierung der Stickstoffmonoxid(NO)-Synthase.

Des weiteren sollen sich die Arzneimittel zur Behandlung bzw. Prophylaxe von Migräne, zur Bekämpfung von Schmerz, insbesondere von chronischem Schmerz und/oder Entzündungsschmerz, zur Behandlung von septischem Schock, von neurodegenerativen Erkrankungen, vorzugsweise multipler Sklerose, Morbus Parkinson, Morbus Alzheimer und/oder Morbus Huntington, Entzündungen, cerebraler Ischämie, Diabetes, Meningitis, Arteriosklerose, zur Wundheilung, zur Tumorbehandlung, zur Inhibierung der Angiogenese oder als Antibiotikum eignen.

Diese Aufgabe wurde durch die Bereitstellung der substituierten Pyrido[1,2-a]pyrimidin-Verbindungen der nachstehenden allgemeinen Formel I gelöst.

Ein Gegenstand der vorliegenden Erfindung sind daher substituierte Pyrido[1,2-a]pyrimidin-Verbindungen der allgemeinen Formel I,

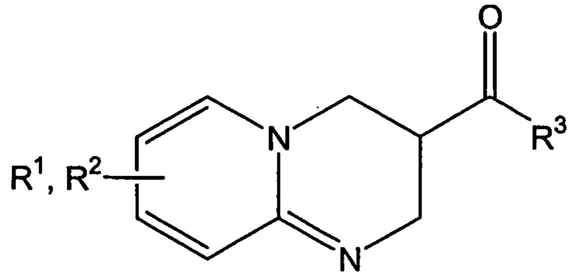

I

worin

$R^1$ und $R^2$, unabhängig voneinander, jeweils für Wasserstoff, Halogen oder einen linearen oder verzweigten, ggf. wenigstens einfach substituierten Alkyl-Rest stehen, und

$R^3$ für einen ggf. wenigstens einfach substituierten, monocyclischen Aryl- oder Heteroaryl-Rest steht, der mit einem ggf. wenigstens einfach substituierten, ggf. wenigstens ein Heteroatom als Ringglied aufweisenden, monocyclischen oder

polycyklischen Ringsystem kondensiert sein kann,
ggf. in Form eines ihrer reinen Stereoisomeren, insbesondere Enantiomeren oder Diastereomeren, ihres Racemates oder in Form einer Mischung von Stereoisomeren, insbesondere der Enantiomeren oder Diastereomeren, in einem beliebigen Mischungsverhältnis, oder jeweils in Form ihrer freien Base oder in Form ihres physiologisch verträglichen Salzes, insbesondere Hydroperchloratsalzes, oder jeweils in Form ihres Solvates, insbesondere Hydrates,
wobei Verbindungen der allgemeinen Formel 1, in denen $R^1$ und $R^2$ für Wasserstoff und einen Methyl-Rest oder $R^1$ und $R^2$ jeweils für Wasserstoff und $R^3$ jeweils für einen unsubstituierten oder einfach mit einem Methyl-Rest, Methoxy-Rest, Cl oder Br substituierten Phenylrest steht sowie die Verbindungen
(3,4-dihydro-9-methyl-2H-pyrido[1,2-a]pyrimidin-3-yl)(3,4-dimethoxyphenyl)-methanon,
(3,4-dihydro-6-methyl-2H-pyrido[1,2-a]pyrimidin-3-yl)(3,4-dimethoxyphenyl)-methanon,
(3,4-dihydro-2H-pyrido[1,2-a]pyrimidin-3-yl)(3,4-dimethoxyphenyl)-methanon und
(3,4-dichlorophenyl)(3,4-dihydro-2H-pyrido[1,2-a]pyrimidin-3-yl)-methanon
sowie jeweils deren Hydroperchloratsalze ausgenommen sind.

Unter einem mono-, bi-, tri- bzw. polycyclischen Ringsystem werden im Sinne der vorliegenden Erfindung mono-, bi-, tri- bzw. polycyclische Kohlenwasserstoffreste verstanden, die gesättigt, ungesättigt oder aromatisch sein können. Sofern ein bi-, tri-oder polycyclisches Ringsystem vorliegt, kann dieses in verschiedenen Ringen auch zwei oder mehr entsprechende Teilstrukturen unterschiedlichen Sättigungsgrades aufweisen. Ggf. kann das mono-, bi-, tri- bzw. polycyclische Ringsystem auch ein oder mehrere Heteroatome als Ringglieder aufweisen, wobei die Ringe jeweils gleiche oder unterschiedliche Heteroatome aufweisen können. Sofern ein bi-, tri-oder polycyclisches Ringsystem vorliegt, sind dessen einzelne Ringe vorzugsweise miteinander kondensiert.

Der Fachmann versteht, daß die Schreibweise $R^1$, $R^2$- in der vorstehenden allgemeinen Formel I, ebenso wie in der nachstehenden allgemeinen Formel II, bedeutet, daß die beiden Reste $R^1$ und $R^2$ beliebig an jeder der vier möglichen Positionen des entsprechenden Rings gebunden sein können.

Bevorzugt sind substituierte Pyrido[1,2-a]pyrimidin-Verbindungen der vorstehenden allgemeinen Formel I, in denen die Reste $R^1$ und $R^2$, unabhängig voneinander, jeweils für H, F, Cl, Br oder einen linearen oder verzweigten, ggf. wenigstens einfach substituierten $C_{1-6}$-Alkyl-Rest, vorzugsweise für H oder einen Methyl-Rest, stehen und $R^3$ die vorstehend genannte Bedeutung hat, ggf. in Form eines ihrer reinen Stereoisomeren, insbesondere Enantiomeren oder Diastereomeren, ihres Racemates oder in Form einer Mischung von Stereoisomeren, insbesondere der Enantiomeren oder Diastereomeren, in einem beliebigen Mischungsverhältnis, oder jeweils in Form ihrer freien Base oder in Form ihres physiologisch verträglichen Salzes, insbesondere Hydroperchloratsalzes, oder jeweils in Form ihres Solvates, insbesondere Hydrates.

Ebenfalls bevorzugt sind substituierte Pyrido[1,2-a]pyrimidin-Verbindungen der vorstehenden allgemeinen Formel I, in denen der Rest $R^3$ für einen ggf. wenigstens einfach substituierten, 5-oder 6-gliedrigen, monocyclischen Aryl- oder Heteroaryl-Rest steht, der mit einem ggf. wenigstens einfach substituierten, ggf. wenigstens ein Heteroatom als Ringglied aufweisenden, mono-, bi- oder tricyclischen Ringsystem kondensiert sein kann, wobei die Ringe des Ringsystems jeweils 5 bis 7-gliedrig sind, vorzugsweise für einen wenigstens einfach substituierten Phenyl-Rest oder einen ggf. wenigstens einfach substituierten Phenyl-, Naphthyl-, Furanyl-, Thiophenyl-, Pyrrolyl-, Imidazolyl-, Pyrazolyl-, Pyridinyl-, Pyrimidinyl-, Chinolinyl- oder Isochinolinyl-Rest steht, und die Reste $R^1$ und $R^2$ jeweils die vorstehend genannte Bedeutung haben, ggf. in Form eines ihrer reinen Stereoisomeren, insbesondere Enantiomeren oder Diastereomeren, ihres Racemates oder in Form einer Mischung von Stereoisomeren, insbesondere der Enantiomeren oder Diastereomeren, in einem beliebigen Mischungsverhältnis, oder jeweils in Form ihrer freien Base oder in Form ihres physiologisch verträglichen Salzes, insbesondere Hydroperchloratsalzes, oder jeweils in Form ihres Solvates, insbesondere Hydrates.

Sofern $R^1$ und/oder $R^2$ der vorstehenden allgemeinen Formel für einen wenigstens einfach substituierten, verzweigten oder unverzweigten Alkyl-Rest steht, können dessen Substituenten jeweils bevorzugt ausgewählt werden aus der Gruppe bestehend aus Halogen und Hydroxy, besonders bevorzugt aus der Gruppe bestehend aus F, Cl, Br und OH. Ist ein Alkyl-Rest mehrfach substituiert, können die Substituenten gleich oder verschieden sein.

Sofern $R^3$ in der vorstehenden allgemeinen Formel I für einen wenigstens einfach substituierten, monocyclischen Aryl- oder Heteroaryl-Rest steht und/oder ein wenigstens einfach substituiertes, mono- oder polycyclisches Ringsystem aufweist, können deren entsprechende Substituenten, jeweils unabhängig voneinander, bevorzugt ausgewählt werden aus der Gruppe bestehend aus Halogen, $C_{1-4}$-Alkoxy, $C_{1-4}$-Alkyl, unsubstituiertem Phenyl und wenigstens einfach substituiertem Phenyl, vorzugsweise aus der Gruppe bestehend aus F, Cl, Br, Methoxy, Ethoxy, Methyl und unsubstituiertem Phenyl. Sofern der Phenylsubstituent selbst einfach oder mehrfach substituiert ist, können dessen Substituenten jeweils vorzugsweise ausgewählt werden aus der Gruppe bestehend aus F, Cl, Br und Methoxy.

Sofern $R^3$ in der vorstehenden allgemeinen Formel I für einen Heteroarylrest steht und/oder ein wenigstens ein Heteroatom als Ringglied enthaltendes, monocyclisches oder polycyklisches Ringsystem aufweist, so können das Heteroatom bzw. die Heteroatome, sofern nicht anders angegeben, jeweils unabhängig voneinander bevorzugt ausgewählt werden aus der Gruppe bestehend aus Stickstoff, Sauerstoff und Schwefel.

Besonders bevorzugt sind substituierte Pyrido[1,2-a]pyrimidin-Verbindungen der vorstehenden allgemeinen

Formel I ausgewählt aus der Gruppe bestehend aus:

3-(2'-Naphthoyl)-3,4-dihydro-2*H*-8-methylpyrido[1,2-a]pyrimidin,
3-(2'-Naphthoyl)-3,4-dihydro-2*H*-6,8-dimethylpyrido[1,2-a]pyrimidin,
3-(4'-Fluorbenzoyl)-3,4-dihydro-2*H*-8-methylpyrido[1,2-a]pyrimidin,
3-Benzoyl-3,4-dihydro-2*H*-6,8-dimethylpyrido[1,2-a]pyrimidin,
3-(4'-Biphenylcarbonyl)-3,4-dihydro-2*H*-8-methylpyrido[1,2-a]pyrimidin,
3-(4'-Biphenylcarbonyl)-3,4-dihydro-2*H*-6-methylpyrido[1,2-a]pyrimidin,
(4-Ethoxy-phenyl)-(8-methyl-3,4-dihydro-2H-pyrido[1,2-a]pyrimidin-3-yl)-methanon,
Biphenyl-4-yl-(6,8-dimethyl-3,4-dihydro-2H-pyrido[1,2-a]pyrimidin-3-yl)-methanon,
(8-Methyl-3,4-dihydro-2H-pyrido[1,2-a]pyrimidin-3-yl)-(6-methyl-naphthalin-2-yl)-methanon, und
(6-Methoxy-naphthalin-2-yl)-(8-methyl-3,4-dihydro-2H-pyrido[1,2-a]pyrimidin-3-yl)-methanon,

ggf. in Form ihrer freien Base oder in Form ihres physiologisch verträglichen Salzes, insbesondere Hydroperchloratsalzes, oder jeweils in Form ihres Solvates, insbesondere Hydrates.

Ein weiterer Gegenstand der vorliegenden Erfindung ist ein Verfahren zur Herstellung der erfindungsgemäßen substituierten Pyrido[1,2-a]pyrimidinVerbindungen der vorstehenden allgemeinen Formel I, in dem wenigstens eine Verbindung der allgemeinen Formel III,

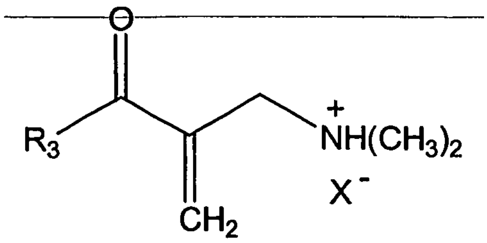

III,

worin $R^3$ die vorstehende Bedeutung hat und $X^-$ für ein Chlorid- oder Bromid-Anion steht, mit wenigstens einer Verbindung der allgemeinen Formel IV,

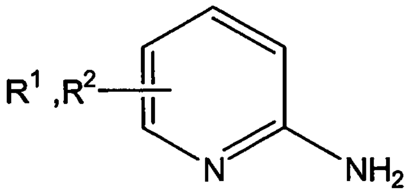

IV,

worin $R^1$ und $R^2$ die vorstehende Bedeutung haben, in einem geeigneten Reaktionsmedium, vorzugsweise in einem Wasser/Alkohol-Gemisch, besonders bevorzugt in einem Wasser/Ethanol-Gemisch, unter Erhitzen, vorzungsweise unter Rückfluß, umgesetzt, und die so erhaltene Verbindung der allgemeinen Formel I ggf. nach üblichen, dem Fachmann bekannten Methoden gereinigt und ggf. isoliert wird.

Die Verbindungen der allgemeinen Formeln III und IV sind käuflich am Markt erhältlich oder können nach üblichen, dem Fachmann bekannten Verfahren hergestellt werden, wie beispielsweise in U. Girreser et al., Synlett 1998, Seiten 263 ff. sowie in Heber, D., Girreser, U., J. Prakt. Chem., 2000, 342, No. 3, Seiten 230-234 beschrieben. Die Herstellung der vorstehend ausgenommenen Verbindungen kann analog zu dem erfindungsgemäßen Verfahren erfolgen und wird ebenfalls in der vorstehend genannten Literatur beschrieben.

Überraschenderweise wurde nun gefunden, daß die substituierten Pyrido [1,2-a]pyrimidin-Verbindungen der

vorstehenden allgemeinen Formel I einschließlich der vorstehend ausgenommenen Verbindungen als Inhibitoren auf die Stickstoffmonoxid-Synthase (NO-Synthase) wirken und sich insbesondere zur Bekämpfung von Schmerz, vorzugsweise chronischem Schmerz und/oder Entzündungsschmerz, zur Prophylaxe und/oder Behandlung von Migräne, zur Behandlung eines septischen Schocks, neurodegenerativer Erkrankungen, vorzugsweise multipler Sklerose, Morbus Parkinson, Morbus Alzheimer und/oder Morbus Huntington, Entzündungen, cerebraler Ischämie, Diabetes, Meningitis, Arteriosklerose, zur Wundheilung, zur Tumorbehandlung, zur Inhibierung der Angiogenese und als Antibiotikum, vorzugsweise durch Auslösung einer unspezifischen Immunantwort gegen Mikroorganismen, eignen.

Ein weiterer Gegenstand der vorliegenden Erfindung ist daher ein Arzneimittel enthaltend wenigstens eine substituierte Pyrido[1,2-a]pyrimidin-Verbindung der allgemeinen Formel II,

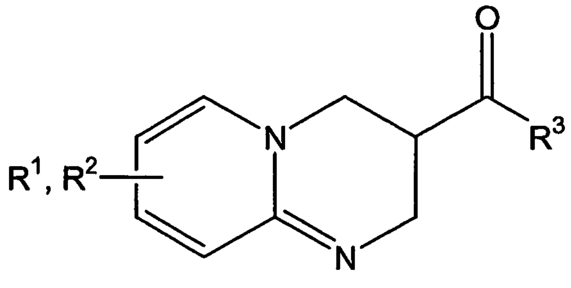

II

worin

$R^1$ und $R^2$, unabhängig voneinander, jeweils für Wasserstoff, Halogen oder einen linearen oder verzweigten, ggf. wenigstens einfach substituierten Alkyl-Rest stehen, und

$R^3$ für einen ggf. wenigstens einfach substituierten, monocyclischen Aryl- oder Heteroaryl-Rest steht, der mit einem ggf. wenigstens einfach substituierten, ggf. wenigstens ein Heteroatom als Ringglied aufweisenden, monocyclischen oder polycyklischen Ringsystem kondensiert sein kann,

ggf. in Form eines ihrer reinen Stereoisomeren, insbesondere Enantiomeren oder Diastereomeren, ihres Racemates oder in Form einer Mischung von Stereoisomeren, insbesondere der Enantiomeren oder Diastereomeren, in einem beliebigen Mischungsverhältnis, oder jeweils in Form ihrer freien Base oder in Form ihres physiologisch verträglichen Salzes, insbesondere Hydroperchloratsalzes, oder jeweils in Form ihres Solvates, insbesondere Hydrates.

Bevorzugt sind Arzneimittel enthaltend wenigstens eine substituierte Pyrido[1,2-a]pyrimidin-Verbindung der vorstehenden allgemeinen Formel 11, worin die Reste $R^1$ und $R^2$, unabhängig voneinander, jeweils für H, F, Cl, Br oder einen linearen oder verzweigten, ggf. wenigstens einfach substituierten $C_{1-6}$-Alkyl-Rest, bevorzugt für H oder Methyl stehen, und $R^3$ die vorstehend genannte Bedeutung hat, ggf. in Form eines ihrer reinen Stereoisomeren, insbesondere Enantiomeren oder Diastereomeren, ihres Racemates oder in Form einer Mischung von Stereoisomeren, insbesondere der Enantiomeren oder Diastereomeren, in einem beliebigen Mischungsverhältnis, oder jeweils in Form ihrer freien Base oder in Form ihres physiologisch verträglichen Salzes, insbesondere Hydroperchloratsalzes, oder jeweils in Form ihres Solvates, insbesondere Hydrates.

Weiterhin bevorzugt sind Arzneimittel enthaltend wenigstens eine substituierte Pyrido[1,2-a]pyrimidin-Verbindung der vorstehenden allgemeinen Formel II, in der $R^3$ für einen ggf. wenigstens einfach substituierten, 5-oder 6-gliedrigen, monocyclischen Aryl- oder Heteroaryl-Rest steht, der mit einem ggf. wenigstens einfach substituierten, ggf. wenigstens ein Heteroatom als Ringglied aufweisenden, mono-, bi- oder tricyclischen Ringsystem kondensiert sein kann, wobei die Ringe des Ringsystems jeweils 5 bis 7-gliedrig sind, vorzugsweise für einen ggf. wenigstens einfach substituierten Phenyl-, Naphthyl-, Furanyl-, Thiophenyl-, Pyrrolyl-, Imidazolyl-, Pyrazolyl-, Pyridinyl-, Pyrimidinyl-, Chinolinyl- oder Isochinolinyl-Rest, besonders bevorzugt für einen ggf. wenigstens einfach substituierten Phenyl- oder Naphtyl-Rest, steht und $R^1$ und $R^2$ jeweils die vorstehend genannte Bedeutung haben, ggf. in Form eines ihrer reinen Stereoisomeren, insbesondere Enantiomeren oder Diastereomeren, ihres Racemates oder in Form einer Mischung von Stereoisomeren, insbesondere der Enantiomeren oder Diastereomeren, in einem beliebigen Mischungsverhältnis, oder jeweils in Form ihrer freien Base oder in Form ihres physiologisch verträglichen Salzes, insbesondere Hydroperchloratsalzes, oder jeweils in Form ihres Solvates, insbesondere Hydrates.

Sofern $R^1$ und/oder $R^2$ der vorstehenden allgemeinen Formel II für einen wenigstens einfach substituierten, verzweigten oder unverzweigten Alkyl-Rest steht, können dessen Substituenten jeweils bevorzugt ausgewählt werden aus der Gruppe bestehend aus Halogen und Hydroxy, besonders bevorzugt aus der Gruppe bestehend aus F, Cl, Br und OH. Ist ein Alkyl-Rest mehrfach substituiert, können die Substituenten gleich oder verschieden sein.

Sofern $R^3$ in der vorstehenden allgemeinen Formel II für einen wenigstens einfach substituierten, monocyclischen Aryl- oder Heteroaryl-Rest steht und/oder ein wenigstens einfach substituiertes, mono- oder polycyclisches Ringsystem aufweist, können deren entsprechende Substituenten, jeweils unabhängig voneinander, bevorzugt ausgewählt werden aus der Gruppe bestehend aus Halogen, $C_{1-4}$-Alkoxy, $C_{1-4}$-Alkyl, unsubstituiertem Phenyl und wenigstens einfach substituiertem Phenyl, vorzugsweise aus der Gruppe bestehend aus F, Cl, Br, Methoxy, Ethoxy, Methyl und unsubstituiertem Phenyl. Sofern der Phenylsubstituent selbst einfach oder mehrfach substituiert ist, können dessen Substituenten jeweils vorzugsweise ausgewählt werden aus der Gruppe bestehend aus F, Cl, Br und Methoxy.

Sofern $R^3$ in der vorstehenden allgemeinen Formel II für einen Heteroarylrest steht und/oder ein wenigstens ein Heteroatom als Ringglied enthaltendes, monocyclisches oder polycyklisches Ringsystem aufweist, so können das Heteroatom bzw. die Heteroatome, sofern nicht anders angegeben, jeweils unabhängig voneinander bevorzugt ausgewählt werden aus der Gruppe bestehend aus Stickstoff, Sauerstoff und Schwefel.

Besonders bevorzugt sind Arzneimittel enthaltend wenigstens eine substituierte Pyrido[1,2-a]pyrimidin-Verbindung der allgemeinen Formel II, ausgewählt aus der Gruppe bestehend aus:

3-(2'-Naphthoyl)-3,4-dihydro-2*H*-8-methylpyrido[1,2-a]pyrimidin,
3-(2'-Naphthoyl)-3,4-dihydro-2*H*-6,8-dimethylpyrido[1,2-a]pyrimidin,
3-(4'-Fluorbenzoyl)-3,4-dihydro-2*H*-8-methylpyrido[1,2-a]pyrimidin,
3-Benzoyl-3,4-dihydro-2*H*-6,8-dimethylpyrido[1,2-a]pyrimidin,
3-(4'-Biphenylcarbonyl)-3,4-dihydro-2*H*-8-methylpyrido[1,2-a]pyrimidin,
3-(4'-Biphenylcarbonyl)-3,4-dihydro-2*H*-6-methylpyrido[1,2-a]pyrimidin,
3-(4'-Brombenzoyl)-3,4-dihydro-2*H*-8-methylpyrido[1,2-a]pyrimidin,
3-(4'-Chlorbenzoyl)-3,4-dihydro-2*H*-8-methylpyrido[1,2-a]pyrimidin,
3-Benzoyl-3,4-dihydro-2*H*-8-methylpyrido[1,2-a]pyrimidin,
(4-Ethoxy-phenyl)-(8-methyl-3,4-dihydro-2H-pyrido[1,2-a]pyrimidin-3-yl)-methanon,
Biphenyl-4-yl-(6,8-dimethyl-3,4-dihydro-2H-pyrido[1,2-a]pyrimidin-3-yl)-methanon,
(8-Methyl-3,4-dihydro-2H-pyrido[1,2-a]pyrimidin-3-yl)-(6-methyl-naphthalin-2-yl)-methanon, und
(6-Methoxy-naphthalin-2-yl)-(8-methyl-3,4-dihydro-2H-pyrido[1,2-a]pyrimidin-3-yl)-methanon,

ggf. in Form ihrer freien Base oder in Form ihres physiologisch verträglichen Salzes, insbesondere Hydroperchloratsalzes, oder jeweils in Form ihres Solvates, insbesondere Hydrates.

Ein weiterer Gegenstand der vorliegenden Erfindung ist die Verwendung wenigstens einer substituierten Pyrido [1,2-a]pyrimidin-Verbindung der vorstehenden allgemeinen Formel II, ggf. in Form eines ihrer reinen Stereoisomeren, insbesondere Enantiomeren oder Diastereomeren, ihres Racemates oder in Form einer Mischung von Stereoisomeren, insbesondere der Enantiomeren oder Diastereomeren, in einem beliebigen Mischungsverhältnis, oder jeweils in Form ihrer freien Base oder in Form ihres physiologisch verträglichen Salzes, insbesondere Hydroperchloratsalzes, oder jeweils in Form ihres Solvates, insbesondere Hydrates, zur Herstellung eines Arzneimittels zur Inhibierung der Stickstoffmonoxid-Synthase, zur Bekämpfung von Schmerz, vorzugsweise von chronischem Schmerz und/oder Entzündungsschmerz, zur Prophylaxe und/oder Behandlung von Migräne, zur Behandlung eines septischen Schocks, neurodegenerativer Erkrankungen, vorzugsweise multipler Sklerose, Morbus Parkinson, Morbus Alzheimer und/oder Morbus Huntington, Entzündungen, cerebraler Ischämie, Diabetes, Meningitis, Arteriosklerose, zur Wundheilung, zur Tumorbehandlung, zur Inhibierung der Angiogenese und als Antibiotikum, vorzugsweise durch Auslösung einer unspezifischen Immunantwort gegen Mikroorganismen.

Die substituierten Pyrido[1,2-a]pyrimidin-Verbindungen der vorstehenden allgemeinen Formeln I und II sowie ggf. jeweils entsprechende Stereoisomere können nach üblichen, dem Fachmann bekannten Verfahren in Form ihrer physiologisch verträglichen Salze erhalten werden, wobei das erfindungsgemäße Arzneimittel eines oder mehrere Salze einer oder mehrerer dieser Verbindungen aufweisen kann.

Die physiologisch verträglichen Salze der substituierten Pyrido[1,2-a]pyrimidinVerbindungen der vorstehenden allgemeinen Formeln I und II können beispielsweise durch Umsetzung mit einer oder mehreren anorganischen oder organischen Säuren, vorzugsweise ausgewählt aus der Gruppe bestehend aus Perchlorsäure, Salzsäure, Bromwasserstoffsäure, Schwefelsäure, Methansulfonsäure, Ameisensäure, Essigsäure, Oxalsäure, Bernsteinsäure, Weinsäure, Mandelsäure, Fumarsäure, Milchsäure, Zitronensäure, Glutaminsäure, Sacharinsäure, Cyclohexansulfamidsäure, Aspartam, Monomethylsebacinsäure, 5-Oxo-prolin, Hexan-1-sulfonsäure, Nicotinsäure, 2-Aminobenzoesäure, 3-Aminobenzoesäure oder 4-Aminobenzoesäure, 2,4,6-Trimethylbenzoesäure, $\alpha$-Liponsäure, Acetylglycin, Acetylsalicylsäure, Hippursäure und Asparaginsäure, erhalten werden.

Bevorzugtes Salz ist das entsprechende Hydroperchloratsalz, das vorzugsweise erhalten werden kann, indem die jeweilige substituierte Pyrido[1,2-a]pyrimidin-Verbindung der vorstehenden allgemeinen Formel I oder II oder ein entsprechendes Stereoisomeres in einem geeigneten organischen Lösungsmittel, wie z.B. Isopropanol, gelöst und mit Perchlorsäure und Wasser in das entsprechende Hydroperchloratsalz übergeführt wird.

Die substituierten Pyrido[1,2-a]pyrimidin-Verbindungen der vorstehenden allgemeinen Formeln I und II sowie

ggf. entsprechende Stereoisomere und jeweils deren physiologisch verträgliche Salze können nach üblichen, dem Fachmann bekannten Verfahren in Form ihrer Solvate, insbesondere Hydrate erhalten werden.

Sofern die substituierten Pyrido[1,2-a]pyrimidin-Verbindungen der vorstehenden allgemeinen Formeln I und II nach ihrer Herstellung in Form einer Mischung ihrer Stereoisomeren, vorzugsweise in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeits-chromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomere, z.B. mittels HPLC an chiraler Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die substituierten Pyrido[1,2-a]pyrimidin-Verbindungen der vorstehenden allgemeinen Formeln I und II und ggf. entsprechende Stereoisomere sowie jeweils die entsprechenden Salze und Solvate sind toxikologisch unbedenklich und eignen sich daher als pharmazeutische Wirkstoffe in Arzneimitteln.

Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder in multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden.

Neben einer oder mehreren der in dem erfindungsgemäßen Arzneimittel zum Einsatz kommenden substituierten Pyrido[1,2-a]pyrimidin-Verbindungen der vorstehenden allgemeinen Formel II, ggf. in Form eines ihrer reinen Stereoisomeren, insbesondere Enantiomeren oder Diastereomeren, ihrer Racemate oder in Form einer Mischung von Stereoisomeren, insbesondere der Enantiomeren oder Diastereomeren, in einem beliebigen Mischungsverhältnis, oder jeweils in Form ihrer freien Base oder in Form ihres physiologisch verträglichen Salzes, insbesondere Hydroperchloratsalzes, oder jeweils in Form ihres Solvates, insbesondere Hydrates, enthält das erfindungsgemäße Arzneimittel üblicherweise weitere physiologisch verträgliche pharmazeutische Hilfsstoffe, die bevorzugt ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays.

Die in dem erfindungsgemäßen Arzneimittel zum Einsatz kommenden substituierten Pyrido[1,2-a]pyrimidin-Verbindungen der vorstehenden allgemeinen Formel II, ggf. in Form eines ihrer reinen Stereoisomeren, insbesondere Enantiomeren oder Diastereomeren, ihrer Racemate oder in Form einer Mischung von Stereoisomeren, insbesondere der Enantiomeren oder Diastereomeren, in einem beliebigen Mischungsverhältnis, oder jeweils in Form ihrer freien Base oder in Form ihrer physiologisch verträglichen Salzes, insbesondere Hydroperchloratsalzes oder jeweils in Form ihres Solvates, insbesondere Hydrates, in einem Depot in gelöster Form oder in einem Pflaster, gegebenenfalls unter Zusatz von die Hautpenetration fördernden Mitteln, sind geeignete perkutane Applikationszubereitungen.

Oral oder perkutan anwendbare Zubereitungsformen können die jeweiligen substituierten Pyrido[1,2-a]pyrimidin-Verbindungen der allgemeinen Formel 11, ggf. in Form eines ihrer reinen Stereoisomeren, insbesondere Enantiomeren oder Diastereomeren, ihrer Racemate oder in Form einer Mischung von Stereoisomeren, insbesondere der Enantiomeren oder Diastereomeren, in einem beliebigen Mischungsverhältnis, oder jeweils in Form ihrer freien Base oder in Form ihres physiologisch verträglichen Salzes, insbesondere Hydroperchloratsalzes, oder jeweils in Form ihres Solvates, insbesondere Hydrates, auch verzögert freisetzen.

Die an den Patienten zu verabreichende Menge der jeweiligen substituierten Pyrido[1,2-a]pyrimidin-Verbindung der allgemeinen Formel II, ggf. in Form eines ihrer reinen Stereoisomeren, insbesondere Enantiomeren oder Diastereomeren, ihres Racemates oder in Form einer Mischung von Stereoisomeren, insbesondere der Enantiomeren oder Diastereomeren, in einem beliebigen Mischungsverhältnis, oder jeweils in Form ihrer freien Base oder in Form ihres physiologisch verträglichen Salzes, insbesondere Hydroperchloratsalzes, oder jeweils in Form ihres Solvates, insbesondere Hydrates, kann variieren und ist beispielsweise abhängig vom Gewicht oder Alter des Patienten sowie von der Applikationsart, der Indikation und dem Schweregrad der Erkrankung. Üblicherweise werden 0.005 bis 500 mg/kg, vorzugsweise 0.05 bis 5 mg/kg Körpergewicht des Patienten wenigstens einer substituierten Pyrido[1,2-a]pyrimidin-Verbindung der allgemeinen Formel II, ggf. in Form eines ihrer reinen Stereoisomeren, insbesondere Enantiomeren oder Diastereomeren, ihrer Racemate oder in Form einer Mischung von Stereoisomeren, insbesondere der Enantiomeren oder Diastereomeren, in einem beliebigen Mischungsverhältnis, oder jeweils in Form ihrer freien Base oder in Form

ihres physiologisch verträglichen Salzes, insbesondere Hydroperchloratsalzes, oder jeweils in Form ihres Solvates, insbesondere Hydrates appliziert.

**Pharmakologische Methoden**

Im folgenden werden die zur Bestimmung der Stickstoffmonoxid-Synthase Inhibierung durch die erfindungsgemäß zum Einsatz kommenden Verbindungen der allgemeinen Formel II verwendeten Assays beschrieben.

**(a) Stickstoffmonoxid-Synthase (NOS) Assay**

Dieser Assay erlaubt die Bestimmung der prozentualen Hemmung von NO-Synthase - im folgenden Enzym genannt - durch eine erfindungsgemäß zum Einsatz kommende Verbindung der allgemeinen Formel II, ggf. in Form eines ihrer reinen Stereoisomeren, insbesondere Enantiomeren oder Diastereomeren, ihrer Racemate oder in Form einer Mischung von Stereoisomeren, insbesondere der Enantiomeren oder Diastereomeren, in einem beliebigen Mischungsverhältnis, oder jeweils in Form ihrer freien Base oder in Form ihrer physiologisch verträglichen Salzes, insbesondere Hydroperchloratsalzes oder jeweils in Form ihres Solvates, insbesondere Hydrates - im folgenden Wirkstoff genannt - mittels Messung der Enzymaktivität bei Einwirken des Wirkstoffs. Dabei wird das Enzym zusammen mit radioaktiv markiertem Arginin und dem jeweiligen Wirkstoff unter geeigneten Bedingungen gemischt. Nach Abbruch der NO-Bildungsreaktion zu einem vorgegebenen Zeitpunkt wird die Menge an nicht umgesetztem Arginin direkt oder indirekt bestimmt. Der Vergleich dieser Menge mit der in einem ohne Zusatz von Wirkstoff und unter sonst gleichen Bedingungen aus der Mischung von Enzym und Arginin zurückbleibenden Menge an Arginin ergibt die prozentuale Hemmung des Enzyms durch den getesteten Wirkstoff.

Dieser Assay läßt sich wie folgt durchführen:

(a) Inkubation des Enzyms mit markiertem Arginin als Substrat in einem Reaktionsgefäß,

(b) Trennung des markierten Arginins von dem gegebenenfalls als Produkt der enzymatischen Reaktion entstandenen, markierten Citrullin zu einem Zeitpunkt, zu dem die Konzentration an Citrullin ansteigt,

(c) Messung der Menge an jeweils abgetrenntem Arginin.

Die Trennung erfolgt über eine Filterplatten-Membran. Dieser Assay eignet sich insbesondere für ein "High Throughput Screening (HTS) auf Mikrotiterplatten (MTP)

**(b) HTS(High-Throughput-Screening)-NOS-Assay:**

In diesem HTS-NOS-Assay wird radioaktives Arginin als Substrat benutzt. Das Assayvolumen kann je nach Art der Mikrotiterplatte (MTP) im Bereich zwischen 25 µl und 250 µl gewählt werden. In Abhängigkeit von der benutzten Enzymquelle werden Cofaktoren und Coenzyme zugefügt. Die Inkubation der Ansätze in dieser Mikrotiterplatte (Assay-MTP) gemäß Schritt (a) wird bei Raumtemperatur vorgenommen und beträgt je nach verwendeter Enzymaktivität (units) zwischen 5 und 60 Minuten. Zum Ende der Inkubation (Schritt (a)) wird die Platte in einen Zellharvester plaziert, der mit einer MTP bestückt ist, die eine Kationenaustauschermembran als Filterboden besitzt (Filter-MTP). Alle Ansätze der Assay-MTP werden in diese Filter-MTP überführt und über eine Kationenaustauscher-Filter-Platte, einen mit Phosphatgruppen beladenen Papierfilter, abgesaugt. Die Filter-MTP wird anschließend mit Puffer oder Wasser gewaschen. Mit Hilfe dieser Vorgehensweise wird das verbliebene Substrat Arginin auf dem Kationenaustauscher gebunden, während das enzymatisch gebildete radioaktive Citrullin quantitativ ausgewaschen wird. Nach Trocknen der Filter-MTP und Zugabe von Szintillationsflüssigkeit (Ready Protein, Fa. Beckmann Coulter GmbH, Krefeld, Deutschland) kann das gebundene Arginin am Szintillationszähler (Packard TRI-CARB Liquid Szintillation Analzer 2000 CA, Fa. Packard Instrument, Meriden, CT 06450, USA) ausgezählt werden. Eine nicht gehemmte Enzym-Reaktion spiegelt sich in einer geringen Radioaktivität wieder. Eine gehemmte Enzymreaktion bedeutet, daß das radioaktive Arginin nicht umgesetzt worden ist. Daraus folgt, auf dem Filter befindet sich dann eine hohe Radioaktivität.

**(c) Formalin-Test an der Ratte**

Die Untersuchungen zur Bestimmung der antinociceptiven Wirkung der erfindungsgemäß zum Einsatz kommenden Verbindungen der allgemeinen Formel II wurden im Formalin-Test an männlichen Ratten (Sprague-Dawley, 150 -170 g, Charles River) durchgeführt.
Im Formalin-Test werden die erste (frühe) Phase (0-15 min nach Formalin-Injektion) und die zweite (späte) Phase (15

- 60 min nach Formalin-Injektion) unterschieden, wie in D. Dubuisson, S.G. Dennis, Pain 4, 161 - 174 (1977) beschrieben. Die frühe Phase stellt als direkte Reaktion auf die Formalin-Injektion ein Modell für Akutschmerz dar, während die späte Phase als Modell für persistierenden (chronischen) Schmerz angesehen wird, wie in T.J. Coderre, J. Katz, A.L. Vaccarino, R. Melzack, Pain, Vol. 52, S. 259, 1993 beschrieben. Die entsprechenden Literaturbeschreibungen werden hiermit als Referenz eingeführt und gelten als Teil der Offenbarung.

Die erfindungsgemäß zum Einsatz kommenden Verbindungen der allgemeinen Formel II wurden in der zweiten Phase des Formalin-Tests untersucht, um Aussagen über Substanzwirkungen im chronisch/entzündlichen Schmerz zu erhalten.

Durch eine einmalige subkutane Formalin-Injektion (50 µl, 5 Gew.-%ig) in die dorsale Seite der rechten Hinterpfote wurde bei freibeweglichen Ratten eine nociceptive Reaktion induziert, die sich in folgenden Verhaltensparametern darstellt: Heben und Halten der betroffenen Pfote (Score 1), Schütteln bzw. Zucken (Score 2), Lecken und Beißen (Score 3). Die aufgrund der Formalininjektion ausgelösten differierenden Verhaltensweisen wurden durch Beobachtung der Ratten in der späten Phase des Formalin-Tests kontinuierlich erfaßt und in einer Bewertung unterschiedlich gewichtet. Normalverhalten, bei dem die Ratte alle vier Pfoten gleichmäßig belastet, wurde als Score 0 registriert. Abhängig von der Applikationsart der erfindungsgemäß zum Einsatz kommenden Verbindungen der allgemeinen Formel II wurde der Applikationszeitpunkt vor der Formalin-Injektion gewählt (intraperitoneal: 15 min, intravenös: 5 min). Nach Injektion von Substanzen, die im Formalin-Test antinozizeptiv wirksam sind, sind die beschriebenen Verhaltensweisen (Score 1 - 3) der Tiere reduziert, evtl. sogar aufgehoben. Der Vergleich erfolgte mit Kontrolltieren, die Vehikel (Lösungsmittel) vor Formalinapplikation erhalten hatten. Das nozizeptive Verhalten wurde als sogenannte Schmerz-Rate (Pain-Rate, PR) berechnet. Die verschiedenen Verhaltensparameter erhielten eine unterschiedliche Gewichtung (Faktor 0, 1, 2, 3). Die Kalkulation erfolgte in Teilintervallen von 3 min nach folgender Formel:

$$PR = [(T_0 \times 0) + (T_1 \times 1) + (T_2 \times 2) + (T_3 \times 3)] / 180,$$

wobei $T_0$, $T_1$, $T_2$, und $T_3$ jeweils der Zeit in Sekunden entspricht, in der das Tier die Verhaltensweisen 0, 1, 2 oder 3 zeigte. Wirkstoff- und Vehikelgruppen umfassen jeweils n = 10 Tiere. Basierend auf den PR-Berechnungen wurde die Wirkung der erfindungsgemäß zum Einsatz kommenden Verbindungen der allgemeinen Formel II als Änderung gegen Kontrolle in Prozent ermittelt. Die $ED_{50}$-Berechnungen erfolgten mittels Regressionsanalyse.

Im folgenden wird die Erfindung anhand von Beispielen erläutert. Diese Erläuterungen sind lediglich beispielhaft und schränken den allgemeinen Erfindungsgedanken nicht ein.

**Beispiele:**

Die verwendeten Chemikalien und Lösungsmittel wurden käuflich bei den üblichen Herstellern erworben (z.B. Fluka, Merck, Acros).

Die NMR-Spektren wurden mit NMR-Spektrometern der Firma Bruker Analytik GmbH, Silberstreifen 4, D-76287 Rheinstetten gemessen. Die Gerätebezeichnungen lauten: für 300 MHz: Avance DPX 300 MHz, für 600 MHz: Avance DRX 600 MHz.

Die ESI-Massenspektren wurden mit einem Gerät vom Typ Finnigan LCQ der Firma Thermoquest (Analystische Systeme GmbH, Boschring 12, D-63329 Egelsbach) gemessen und mit der Software Xcalibur ausgewertet.

**Beispiel 1:**

3-(2'-Naphthoyl)-3,4-dihydro-2*H*-8-methylpyrido[1,2-a]pyrimidinium-Perchlorat

1 mmol (255 mg) 1-(2'-Naphtyl)-2-(dimethylaminomethyl)prop-2-en-1-on-Hydrochlorid und 1 mmol (108 mg) 2-Amino-4-methylpyridin (2-Amino-4-picolin) wurden in 15 ml Wasser/Ethanol (1:1 Volumen/Volumen) gelöst und 1 Stunde unter Rückfluß erhitzt. Das Lösungsmittel wurde im Vakuum entfernt (Badtemperatur 50°C), der so erhaltene Rückstand in 20 ml 2-Propanol gelöst, unter Kühlung (Eis/Wasser/Kochsalz) mit 4 mmol 70 Vol.-%iger Perchlorsäure versetzt und 30 min bei 0 bis -10°C gerührt. Der ausfallende Niederschlag wurde abgesaugt und 30 min in 10 ml 2-Propanol unter Rückfluß erhitzt. Der dann beim Abkühlen ausfallende Niederschlag wurde filtriert, aus Methanol umkristallisiert und im Ölpumpenvakuum bei 60°C getrocknet.

Ausbeute: 217 mg (0.54 mmol) 54% der Theorie, hellgelbe Kristalle,
Schmelzpunkt.: 186°C
$C_{20}H_{19}N_2O_5Cl$ (402.83)
Berechnet: C 59.63 H 4.75 N 6.96

Gefunden: C 59.61 H 4.85 N 6.90
$^{1}H$-NMR (300 MHz, DMSO-$d_6$):

δ/ppm (TMS) = 2.32 (s, br, 3H, $CH_3$), 3.62 (mc, 1 H, H-2/4), 3.84 (d, 1H, $^{2}J$ = 13.9, H-2/4), 4.41-4.61 (m, 3H, H-3, H-2/4), 6.74 (d, 1 H, $^{2}J$ = 6.9, ArH), 6.81 (s, 1 H, ArH), 7.69 (mc, 2H, ArH), 7.92 (d, 1 H, $^{3}J$ = 6.9, ArH), 7.99-8.15 (m, 5H, ArH), 8.86 (s, 1 H, ArH), 9.31 (s, br, NH).

MS (El, MeOH):

m/z (%) = 302 ($M^+$der Base, 21), 301 ($M^+$-1, 14), 285 (3), 271 (2), 259 (2), 233 (2), 210 (11), 194 (7), 182 (4), 172 (7), 155 (20), 147 ($M^+$-2'-Naphthoyl, 100), 127 (41), 121 (14), 109 (31), 92 (26), 77 (9), 65 (18), 44 (24).

**Beispiel 2:**

3-(2'-Naphthoyl)-3,4-dihydro-2*H*-6,8-dimethylpyrido[1,2-a]pyrimidinium-Perchlorat

1 mmol (255 mg) 1-(2'-Naphthyl)-2-(dimethylaminomethyl)prop-2-en-1-on-Hydrochlorid und 1 mmol (122 mg) 2-Amino-4,6-dimethylpyridin wurden in 5 ml Wasser/ Ethanol (1:1 Volumen/Volumen) gelöst und 1.5 Stunden unter Rückfluß erhitzt. Das Lösungsmittel wurde im Vakuum entfernt (Badtemperatur 50°C), der Rückstand in 5 ml 2-Propanol gelöst, unter Kühlung (Eis/Wasser/ Kochsalz) mit einer Mischung von 70 Vol-%iger Perchlorsäure und 2-Propanol (1:1 Volumen/Volumen) gefällt und 30 min im Eisbad gerührt. Der so erhaltene hellgelbe Niederschlag wurde abgesaugt, mit wenig 2-Propanol gewaschen und im Ölpumpenvakuum getrocknet (24 Stunden, 40°C).
Ausbeute: 150 mg (0.36 mmol), 36% der Theorie, hellgelbe Kristalle, $C_{21}H_{21}N_2O_5Cl$ (416.86)
$^{1}H$-NMR (300 MHz, DMSO-$d_6$):

δ/ppm (TMS) = 2.5* (s, br, 3H, $CH_3$), 3.42-3.65 (m, 1H, H-2/4), 3.75-3.85 (m, 1H, H-2/4), 4.32-4.55 (m, 3H, H-3, H-2/4), 6.70 (s, 1 H, ArH, H-7/9), 6.76 (s, 1 H, ArH, H-7/9), 7.72 (mc, 2H, ArH), 7.99-8.17 (m, 4H, ArH), 8.90 (s, 1 H, ArH), 9.54 (s, br, NH). (*) Verdeckt durch DMSO-Signal.

**Beispiel 3:**

3-(4'-Brombenzoyl)-3,4-dihydro-2*H*-8-methylpyrido[1,2-a]pyrimidinium-Perchlorat

1 mmol (305 mg) 1-(4'-Bromphenyl)-2-(dimethylaminomethyl)prop-2-en-1-on-Hydrochlorid und 1 mmol (108 mg) 2-Amino-4-methylpyridin (2-Amino-4-picolin) wurden in 5-10 ml Wasser/ Ethanol (1:1 Volumen/Volumen) gelöst und 1 Stunde unter Rückfluß erhitzt. Das Lösungsmittel wurde im Vakuum entfernt (Badtemperatur 50°C), der Rückstand in 10 ml 2-Propanol gelöst, unter Kühlung mit 4 mmol 70 Vol.-%iger Perchlorsäure versetzt und 30 min im Eisbad gerührt und das Lösungsmittel im Vakuum entfernt. Der ölige Rückstand wurde mit wenig Ethanol 1 Stunde bei 0°C gerührt und der so erhaltene farblose Niederschlag wurde abgesaugt und im Ölpumpenvakuum bei 60°C getrocknet.
Ausbeute: 198 mg (0.46 mmol), 46% der Theorie, farblose Kristalle,
Schmelzpunkt: 158-161°C
$C_{16}H_{16}N_2O_5BrCl$ (431.67)
Berechnet: C 44.52 H 3.74 N 6.49
Gefunden: C 44.48 H 3.83 N 6.39
$^{1}H$-NMR (300 MHz, DMSO-$d_6$):

δ/ppm (TMS) = 2.31 (s, 3H, $CH_3$), 3.51 (mc, 1 H, H-2/4), 3.75 (d, 1 H, $^{2}J$ = 12.6, H-2/4), 4.35-4.51 (m, 3H, H-3, H-2/4), 6.72-6.76 (m, 2H, H-7/9), 7.81 (d, 2H, $^{3}J$ = 8.7, H-3'/5'), 7.91 (d, 1 H, $^{3}J$ = 6.8, H-6), 7.98 (d, 2H, $^{3}J$ = 8.7, H-2'/6'), 9.20 (s, br, NH).

MS (El, MeOH):

m/z (%) = 332/330 ($M^+$der Base, 6), 330/328 ($M^+$-1, 11), 315 (2), 287 (2), 262/260 (1), 240/238 (1), 202/200 (6), 185/183 (4'-Brombenzoyl, 16), 175 (3), 157/155 (11), 147 ($M^+$-4'-Brombenzoyl, 100), 121 (11), 108 (8), 93 (15), 80 (7), 76 (11), 65 (18), 50 (13), 44 (46).

**Beispiel 4:**

3-(4'-Fluorbenzoyl)-3,4-dihydro-2*H*-8-methylpyrido[1,2-a]pyrimidinium-Perchlorat

1 mmol (243 mg) 1-(4'-Fluorphenyl)-2-(dimethylaminomethyl)prop-2-en-1-on-Hydrochlorid und 1 mmol (108 mg) 2-Amino-4-methylpyridin (2-Amino-4-picolin) wurden in 5 ml Wasser/ Ethanol (1:1 Volumen/Volumen) gelöst und 1 Stunde unter Rückfluß erhitzt. Das Lösungsmittel wurde im Vakuum entfernt (Badtemperatur 50°C), der Rückstand in 5 ml 2-Propanol gelöst, unter Kühlung mit 4 mmol 70 Vol.-%iger Perchlorsäure versetzt und 30 min im Eisbad gerührt und das Lösungsmittel im Vakuum entfernt. Der ölige Rückstand wurde nochmals mit 5 ml 2-Propanol aufgenommen und zum Sieden erhitzt. Der beim Abkühlen ausfallende Niederschlag wurde abgesaugt und im Ölpumpenvakuum getrocknet (12 Stunden, 40°C).

Ausbeute: 53 mg (0.14 mmol), 14% der Theorie, gelbe Kristalle/gelbes Öl, $C_{16}H_{16}N_2O_5FCl$ (370.76)

$^1$H-NMR (300 MHz, DMSO-$d_6$):

δ/ppm (TMS) = 2.31 (s, 3H, $CH_3$), 3.50 (mc, 1 H, H-2/4), 3.73 (mc, 1 H, H-2/4), 4.28-4.52 (m, 3H, H-3, H-2/4), 6.72-6.76 (m, 2H, H-7/9), 7.40 (mc, 2H, ArH), 7.90 (mc, 1 H, H-6), 8.18 (mc, 2H, ArH), 9.22 (s, br, NH).

MS (EI, MeOH):

m/z (%) = 271 ($M^+$+1 der Base, 1), 270 ($M^+$, 5), 269 ($M^+$-1, 4), 254 (1), 239 (3), 222 (4), 195 (3), 90 (6), 181 (2), 155 (4), 147 ($M^+$-4'-Fluorbenzoyl, 42), 135 (6), 127 (6), 123 (4'-Fluorbenzoyl, 34), 121 (10), 108 (28), 95 (34), 80 (28), 65 (16), 58 (100), 44 (52).

**Beispiel 5:**

3-Benzoyl-3,4-dihydro-2*H*-8-methylpyrido[1,2-a]pyrimidiniumium-Perchlorat

1 mmol (225.7 mg) 1-Phenyl-2-(dimethylaminomethyl)prop-2-en-1-on-Hydrochlorid und 1 mmol (108 mg) 2-Amino-4-methylpyridin (2-Amino-4-picolin) wurden in 5 ml Ethanol/Wasser (1/1 Volumen/Volumen) gelöst und 1 Stunde unter Rückfluß erhitzt. Das Lösungsmittel wurde im Vakuum entfernt (Badtemperatur 50°C), der Rückstand in 3 ml 2-Propanol gelöst und gekühlt (Eis/Wasser/ Natriumchlorid). Anschließend wurde in der Kälte durch tropfenweise Hinzufügen einer Mischung von 70 Vol.-%iger Perchlorsäure und 2-Propanol (1/1 Volumen/Volumen) ein Niederschlag durch Fällung erhalten und 30 min im Eisbad gerührt. Die überstehende Lösung wurde entfernt und der Niederschlag 30 min in 10 ml 2-Propanol bei 0°C gerührt, die überstehende Lösung dekantiert, aus Methanol umkristallisiert und der Rückstand im Ölpumpenvakuum bei 60°C getrocknet.

Ausbeute.: 204 mg (0.58 mmol) 58% der Theorie, hellgelbe Kristalle,
Schmelzpunkt: > 290°C
$C_{16}H_{17}N_2O_5Cl$ (352.77)
Berechnet: C 54.47 H 4.86 N 7.94
Gefunden: C 54.65 H 4.95 N 7.79
$^1$H-NMR (300 MHz, DMSO-$d_6$):

δ/ppm (TMS) = 2.29 (s, br, 3H, $CH_3$), 3.51 (mc, 1 H, H-2/4), 3.74 (mc, 1 H, H-2/4), 4.34-4.51 (m, 3H, H-3, H-2/4), 6.71 (dd, 1 H, $^3J$ = 6.8, $^4J$ = 1.7, H-7), 6.79 (s, 1H, H-9), 7.57 (mc, 2H, H-3'/5'), 7.70 (t, 1 H, $^3J$ = 7.4, H-4'), 7.90 (d, 1H, $^3J$ = 6.8, H-6), 8.04 (d, 2H, $^3J$ = 7.3, H-2'/6'), 9.38 (s, br, NH).

MS (EI, MeOH):

m/z (%) = 252 ($M^+$der Base, 21), 251 ($M^+$-1, 1), 188 (3), 177 (2), 172 (17), 160 (1), 147 ($M^+$-Benzoyl, 7), 135 (2), 131 (2), 121 (2), 112 (3), 108 (31), 105 (Benzoyl, 14), 92 (2), 80 (24), 77 (14), 66 (4), 58 (100), 51 (7), 44 (32), 42 (13).

**Beispiel 6:**

3-Benzoyl-3,4-dihydro-2*H*-6,8-dimethylpyrido[1,2-a]pyrimidinium-Perchlorat

1 mmol (225.7 mg) 1-Phenyl-2-(dimethylaminomethyl)prop-2-en-1-on-Hydrochlorid und 1 mmol (122 mg) 2-Amino-4,6-dimethylpyridin wurden in 5 ml Ethanol/Wasser (1/1 Volumen/Volumen) gelöst und 1 Stunde unter Rückfluß erhitzt. Das Lösungsmittel wurde im Vakuum entfernt (Badtemperatur 50°C), der Rückstand in 3 ml 2-Propanol gelöst

und gekühlt (Eis/Wasser/ Natriumchlorid). Anschließend wurde in der Kälte durch tropfenweise Hinzufügen einer Mischung von 70 Vol.-%iger Perchlorsäure und 2-Propanol (1/1 Volumen/Volumen) ein Niederschlag durch Fällung erhalten und 30 min im Eisbad gerührt. Die überstehende Lösung wurde entfernt und der Niederschlag 30 min in 10 ml 2-Propanol bei 0°C gerührt, die überstehende Lösung dekantiert, aus Methanol umkristallisiert und der Rückstand im Ölpumpenvakuum bei 40°C getrocknet.

Ausbeute: 120 mg (0.32 mmol) 32% der Theorie, farblose Kristalle,
Schmelzpunkt: 78°C
$C_{17}H_{19}N_2O_5Cl$ (366.80)
Berechnet: C 55.67 H 5.22 N 7.63
Gefunden: C 55.83 H 5.31 N 7.50
$^1$H-NMR (300 MHz, DMSO-$d_6$):

δ/ppm (TMS) = 2.27 (s, br, 3H, $CH_3$), 2.47 (s, br, 3H, $CH_3$), 3.41-3.47 (m, 1 H, H-2/4), 3.73-3.82 (m, 1 H, H-2/4), 4.29-4.42 (m, 3H, H-3, H-2/4), 6.69 (s, br, 2H, H-7/9), 7.62 (mc, 2H, H-3'/5'), 7.73 (mc, 1 H, H-4'), 8.08 (d, 1 H, $^3$J = 8.4, H-2'/6'), 9.28 (s, br, NH).

MS (El, MeOH):

m/z (%) = 267 ($M^+$+1 der Base, 1), 266 ($M^+$, 6), 265 ($M^+$-1, 8), 236 (2), 223 (2), 197 (2), 176 (6), 172 (13), 161 ($M^+$-Benzoyl, 66), 149 (10), 135 (13), 122 (34), 112 (2), 105 (Benzoyl, 56), 94 (15), 91 (11), 77 (60), 65 (5), 58 (100), 51 (21), 44 (52).

**Beispiel 7:**

3-(4'-Chlorbenzoyl)-3,4-dihydro-2*H*-8-methylpyrido[1,2-a]pyrimidinium-Perchlorat

1 mmol (260 mg) 1-(4'-Chlorphenyl)-2-(dimethylaminomethyl)prop-2-en-1-on-Hydrochlorid und 1 mmol (108 mg) 2-Amino-4-methylpyridin (2-Amino-4-picolin) wurden in 5 ml Ethanol/ Wasser (1/1 Volumen/Volumen) gelöst und 1 Stunde unter Rückfluß erhitzt. Das Lösungsmittel wurde im Vakuum entfernt (Badtemperatur 50°C), der Rückstand in 3 ml 2-Propanol gelöst und gekühlt (Eis/ Wasser/ Natriumchlorid). Anschließend wurde in der Kälte durch tropfenweise Hinzufügen einer Mischung von 70 Vol.-%iger Perchlorsäure und 2-Propanol (1/1 Volumen/Volumen) ein Niederschlag durch Fällung erhalten und 30 min im Eisbad gerührt. Die überstehende Lösung wurde entfernt und der Niederschlag 30 min in 10 ml 2-Propanol bei 0°C gerührt, die überstehende Lösung dekantiert, aus Methanol umkristallisiert und der Rückstand im Ölpumpenvakuum bei 60°C getrocknet.

Ausbeute: 186 mg (0.47 mmol), 47% der Theorie, hellgelbe Kristalle,
Schmelzpunkt: 123-125°C
$C_{16}H_{16}N_2O_5Cl_2$ (387.22)
Berechnet: C 49.63 H 4.17 N 7.23
Gefunden: C 49.77 H 4.26 N 7.11
$^1$H-NMR (300 MHz, DMSO-$d_6$):

δ/ppm (TMS) = 2.31 (s, br, 3H, $CH_3$), 3.52 (mc, 1H, H-2/4), 3.73-3.81 (m, 1H, H-2/4), 4.35-4.81 (m, 3H, H-3, H-2/4), 6.74 (d, 1H, $^2$J = 6.9, H-7), 6.78 (s, 1H, H-9), 7.67 (d, 2H, 3J = 8.6, H-3'/5'), 7.90 (d, 1H, $^3$J = 6.9, H-6), 8.08 (d, 2H, 3J =8.6, H-2'/6'), 9.27 (s, br, NH).

MS (El, MeOH):

m/z (%) = 287 ($M^+$+1 der Base, 4), 286 ($M^+$,9), 285 ($M^+$-1,13), 271 (2), 257 (2), 249 (1), 181 (2),175 (3), 166(3), 156 (7), 147 ($M^+$-Chlorbenzoyl, 100), 145 (11), 141 (16), 139 (Chlorbenzoyl, 48); 133 (6), 121 (12),111 (29), 92 (26), 75 (15), 65 (21), 58 (11), 44 (34).

**Beispiel 8:**

3-(4'-Biphenylcarbonyl)-3,4-dihydro-2*H*-8-methylpyrido[1,2-a]pyrimidinium-Perchlorat

1 mmol (302 mg) 1-Biphenyl-2-(dimethylaminomethyl)prop-2-en-1-on-Hydrochlorid und 1 mmol (108 mg) 2-Amino-4-methylpyridin (2-Amino-4-picolin) wurden in 5 ml Wasser/ Ethanol (1/1 Volumen/Volumen) gelöst und 1 Stunde unter Rückfluß erhitzt. Das Lösungsmittel wurde im Vakuum entfernt (Badtemperatur 50°C), der Rückstand in 8 ml 2-Propanol gelöst und gekühlt (Eis/Wasser/ Natriumchlorid). Anschließend wurde in der Kälte durch tropfenweise Hinzufügen einer Mischung von 70 Vol.-%iger Perchlorsäure und 2-Propanol (1/1 Volumen/Volumen) ein Niederschlag durch Fällung erhalten und 30 min im Eisbad gerührt. Die überstehende Lösung wurde entfernt und der Niederschlag 30 min in 10 ml 2-Propanol bei 0°C gerührt, die überstehende Lösung dekantiert, aus Methanol umkristallisiert und der Rückstand im Ölpumpenvakuum bei 60°C getrocknet.

Ausbeute: 258 mg (0.60 mmol) 60% der Theorie, hellgelbe Kristalle,
Schmelzpunkt: 111°C
$C_{22}H_{21}N_2O_5Cl$ (428.87)
Berechnet: C 61.61 H 4.93 N 6.53
Gefunden: C 61.73 H 5.11 N 6.52
$^1$H-NMR (300 MHz, DMSO-$d_6$):

δ/ppm (TMS) = 2.33 (s, br, 3H, $CH_3$), 2.84-2.88 (m, 1 H, H-2/4), 3.59 (mc, 1 H, H-2/4), 4.39-4.56 (m, 3H, H-3, H-2/4), 6.67-6.78 (m, 2H, H-7, H-9), 7.43-7.56 (m, 3H, H-4", H-3"/5"), 7.78 (d, 2H, $^3J$ = 7.0, H-2"/6"), 7.92 (d, 2H, $^3J$ = 8.5, H-3'/5'), 7.94 (d, 1 H, $^3J$ = 6.8, H-6), 8.16 (d, 2H, $^3J$ = 8.5, H-2'/6'), 9.20 (s, br, NH).

**Beispiel 9:**

3-(4'-Biphenylcarbonyl)-3,4-dihydro-2*H*-6-methylpyrido[1,2-a]pyrimidinium-Perchlorat

1 mmol (302 mg) 1-Biphenyl-2-(dimethylaminomethyl)prop-2-en-1-on-Hydrochlorid und 1 mmol (108 mg) 2-Amino-6-methylpyridin (2-Amino-6-picolin) wurden in 5 ml Wasser/ Ethanol (1/1 Volumen/Volumen) gelöst und 1 Stunde unter Rückfluß erhitzt. Das Lösungsmittel wurde im Vakuum entfernt (Badtemperatur 50°C), der Rückstand in 3 ml 2-Propanol gelöst und gekühlt (Eis/Wasser/ Natriumchlorid). Anschließend wurde in der Kälte durch tropfenweise Hinzufügen einer Mischung von 70 Vol.-%iger Perchlorsäure und 2-Propanol (1/1 Volumen/Volumen) ein Niederschlag durch Fällung erhalten und 30 min im Eisbad gerührt. Die überstehende Lösung wurde entfernt und der Niederschlag 30 min in 10 ml 2-Propanol bei 0°C gerührt, die überstehende Lösung dekantiert, aus Methanol umkristallisiert und der Rückstand im Ölpumpenvak. bei 60°C getrocknet.

Ausbeute: 156 mg (0.36 mmol) 36% der Theorie, hellgelbe Kristalle,
$C_{22}H_{21}N_2O_5Cl$ (428.87)
Berechnet: C 61.61 H 4.93 N 6.53
Gefunden: C 61.82 H 5.02 N 6.38
$^1$H-NMR (300 MHz, DMSO-$d_6$):

δ/ppm (TMS) = 2.5* (s, br, 3H, $CH_3$), 3.51 (mc, 1H, H-2/4), 3.79 (mc, 1H, H-2/4), 4.354.55 (m, 3H, H-3, H-2/4), 6.81 (d, 1H, $^3J$ = 7.0, H-7(9), 6.95 (d,1 1H, $^3J$ = 9.0, H-7/9), 7.42 7.55 (m, 3H, H-3"/5", H-4"), 7.70 (mc, 1H, H-8), 7.79 (d, 2H, $^3J$ = 7.0, H-2"/6"), 7.90 (d, 1H, $^3J$ = 8.4, H-3'/5'),8.18 (d, 2H, $^3J$=8.4, H-2'/6'), 9.60 (s, br, NH). (*) zT. verdeckt durch DMSO-Signal.

MS (EI, MeOH):

m/z(%) = 329 ($M^+$+1 der Base, 2), 328 ($M^+$, 8), 327 ($M^+$-1, 8), 313 (1), 300 (1), 285 (2), 266 (1), 248 (2), 236 (3), 222 (1), 208 (5),181 (4'-Biphenylcarbonl, 16), 167(2), 152(22), 147($M^+$-4'-Biphenylcarbonyl, 100),121 (13), 108(11), 92(23),80(6),76(5),65(16),58(10),44(11).

Die folgenden Beispielverbindungen wurden analog gemäß den vorstehend beschriebenen Beispielen hergestellt.

**Beispiel 10:**

(4-Ethoxy-phenyl)-(8-methyl-3,4-dihydro-2H-pyrido[1,2-a]pyrimidin-3-yl)-methanon-Hydroperchlorat

**Beispiel 11:**

Biphenyl-4-yl-(6,8-dimethyl-3,4-dihydro-2H-pyrido[1,2-a]pyrimidin-3-yl)-methanon-Hydroperchlorat

**Beispiel 12:**

(8-Methyl-3,4-dihydro-2H-pyrido[1,2-a]pyrimidin-3-yl)-(6-methyl-naphthalin-2-yl)-methanon-Hydroperchlorat

**Beispiel 13:**

(6-Methoxy-naphthalin-2-yl)-(8-methyl-3,4-dihydro-2H-pyrido[1,2-a]pyrimidin-3-yl)-methanon-Hydroperchlorat

**Pharmakologische Daten**

Die NOS- und HTS-NOS-Assays wurden wie vorstehend beschrieben durchgeführt.

Enzympräparation

Als Ausgangsgewebe wurden Ratten-Cerebelli benutzt. Die Tiere wurden betäubt und getötet, das Gehirngewebe, das Cerebellum, wurde herauspräpariert, pro Rattenkleinhirn wurde 1 ml Enzympräparationspuffer (4°C) hinzugegeben, und es wurde mit einem Polytron-Homogenisierer für 1 min bei 6000 U/min aufgeschlossen. Danach erfolgte Zentrifugation bei 4°C für 15 min bei 20.000 g und anschließend Abdekantieren und portioniertes Einfrieren des Überstandes bei -80 °C, der Niederschlag wurde verworfen. Der gefrorene Überstand wurde später für das Assay aufgetaut und auf eine Mikrotiterplatte pipettiert.

Inkubationsansatz:

Verwendet wurden 96-well MTP mit einer "Well"-Kapazität von < 250 µl. Die Pipettierreihenfolge ist in der nachfolgenden Tabelle 1 wiedergegeben:

**Tabelle 1:**

| Substanz | Molarität i.A. | Volumen | *Protein i.A: |
|---|---|---|---|
| 1. Inkubat.-Puffer | - | 100 µl | - |
| 2. zu prüfende Verbindung gemäß Beispiel 1-9 | variabel; vorzugsweise $10^{-5}$M | variabel; vorzugsweise 20 µl | - |
| 3. NADPH | 0,5 mM | 20 µl | - |
| 4. Enzym | - | variabel; maximales Volumen der Enzymlösung 50 µl | variabel; maximale einsetzbare Proteinmenge = 100 µg |
| 5. [$^3$H]Substrat | variabel; vorzugsweise 50 nM | variabel; vorzugsweise 10 µl | - |
| Endvolumen: | | max. 250 µl | |

* Die Proteinbestimmung erfolgte nach O.H. Lowry et al; J. Biol.Chem. 193, 265 (1951). Die entsprechende Literaturbeschreibung wird hiermit als Referenz eingeführt und gilt als Teil der Offenbarung.

i.A. = im Ansatz

Nach beendetem Pipettiervorgang wurde ein Deckel auf diese MTP (Assay-MTP) gelegt und bei 25°C (Raumtemperatur [RT]) für 5 - 60 min, je nach Menge und Aktivität des eingesetzten Enzyms, inkubiert (siehe hierzu Tabelle 1). Anschließend wurde der Inhalt der Assay-MTP mit Hilfe eines 96-well Cell-Harvesters in eine 96-well Kationenaustauscher MTP (Filter-MTP) transferiert, abgesaugt und einmalig mit 200 ml $H_2O$ gewaschen.

Dann wurde die Platte für 1 Stunde bei 60 °C im Trockenschrank getrocknet und anschließend wurde die Bodenseite der Filter-MTP von unten her exakt mit einem "back seal" versiegelt. Danach wurden pro Weil 35 μl Szintillator hinzupipettiert. Ferner wurde die Plattenoberseite mit einem "top seal" versiegelt. Nach 1 h Wartezeit wurde die Platte am β-Counter ausgemessen.

Im HTS-Betrieb wurden das Inkubationsmedium, NADPH- und Enzymlösung vor Beginn des Pipettierschrittes vereint, um nicht zeitaufwendig drei separate Pipettierungen vornehmen zu müssen.

Verwendete Materialien

Arginin, L-[2, 3, 4-$^3$H]-monohydrochlorid; Best.-Nr. NET-1123, Fa. NEN $CaCl_2$ wasserfrei; Best.- Nr. 2388.1000; Fa. Merck KGaA

1.4-Dithiothreitol (DTT), Best.-Nr. 708984; Fa. ROCHE

$Na_2$EDTA-Dihydrat; Best.-Nr. 03680; Fa. FLUKA

HEPES, Best:-Nr. H-3375; Fa. SIGMA

NADPH, Tetranatriumsalz; Best.-Nr. 1585363; Fa. ROCHE

TRIS; BEST.-Nr. 93349; Fa. FLUKA

Enzym-Präparationspuffer:

50 mM Tris-HCl mit 1 mM EDTA:

Der pH-Wert des Puffers wurde bei 4 °C auf 7,4 eingestellt.

Inkubationspuffer (-medium):

50 mM HEPES mit 1 mM EDTA; 1,25 mM $CaCl_2$ und 1 mM Dithiothreitol.

Der pH-Wert des Puffers wurde bei 25 °C auf 7,4 eingestellt.

Waschmedium: $H_2O$

In der folgenden Tabelle 2 wird die Hemmung der Enzymaktivität durch die jeweilige substituierte Pyrido[1,2-a]pyrimidin-Verbindung gemäß Beispiel 1-9 als $IC_{50}$ dargestellt, der sog. Inhibitory Concentration 50, die ausdrückt, bei welcher Konzentration 50% der Aktivität des Enzyms unterdrückt werden.

**Tabelle 2:**

| Verbindung gemäß | NOS Arg $IC_{50}$ (μM) |
|---|---|
| Beispiel 1: | 1,4 |
| Beispiel 2: | 6,7 |
| Beispiel 3: | 14 |
| Beispiel 4: | 8,5 |
| Beispiel 5: | 9,1 |
| Beispiel 6: | 8,4 |
| Beispiel 7: | 6,4 |
| Beispiel 8: | 13 |
| Beispiel 9: | 7,6 |

**Formalin Test an der Ratte:**

Die analgetische Wirkung der erfindungsgemäß zum Einsatz kommenden Verbindungen der allgemeinen Formel II wurde wie vorstehend beschrieben ermittelt. Die untersuchten Verbindungen zeigten jeweils eine mittelstarke bis starke Hemmung der durch Formalin induzierten Nozizeption.

In der nachfolgenden Tabelle ist der Wert für die substituierte Pyrido[1,2-a]pyrimidin-Verbindung gemäß Beispiel 1 wiedergegeben:

**Tabelle 3:**

| Verbindung gemäß | $ED_{50}$ |
|---|---|
| 1 | 7,27 mg/kg i.v. |

## Patentansprüche

1. Substituierte Pyrido[1,2-a]pyrimidin-Verbindungen der allgemeinen Formel I

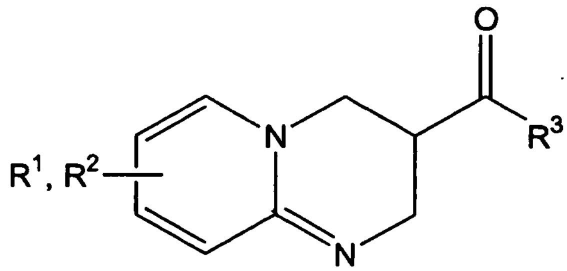

I

worin
$R^1$ und $R^2$, unabhängig voneinander, jeweils für Wasserstoff, Halogen oder für einen linearen oder verzweigten, ggf. wenigstens einfach substituierten Alkyl-Rest stehen, und
$R^3$ für einen ggf. wenigstens einfach substituierten, monocyclischen Aryl- oder Heteroaryl-Rest steht, der mit einem ggf. wenigstens einfach substituierten, ggf. wenigstens ein Heteroatom als Ringglied aufweisenden, monocyclischen oder polycyklischen Ringsystem kondensiert sein kann,
ggf. in Form eines ihrer reinen Stereoisomeren, insbesondere Enantiomeren oder Diastereomeren, ihres Racemates oder in Form einer Mischung von Stereoisomeren, insbesondere der Enantiomeren oder Diastereomeren, in einem beliebigen Mischungsverhältnis, oder jeweils in Form ihrer freien Base oder in Form ihres physiologisch verträglichen Salzes, insbesondere Hydroperchloratsalzes, oder jeweils in Form ihres Solvates, insbesondere Hydrates,
wobei Verbindungen der allgemeinen Formel I, in denen $R^1$ und $R^2$ für Wasserstoff und einen Methyl-Rest oder $R^1$ und $R^2$ jeweils für Wasserstoff und $R^3$ jeweils für einen unsubstituierten oder einfach mit einem Methyl-Rest, Methoxy-Rest, Cl oder Br substituierten Phenylrest steht sowie die Verbindungen (3,4-dihydro-9-methyl-2H-pyrido[1,2-a]pyrimidin-3-yl)(3,4-dimethoxyphenyl)-methanon,
(3,4-dihydro-6-methyl-2H-pyrido[1,2-a]pyrimidin-3-yl)(3,4-dimethoxyphenyl)-methanon,
(3,4-dihydro-2H-pyrido[1,2-a]pyrimidin-3-yl)(3,4-dimethoxyphenyl)-methanon und
(3,4-dichlorophenyl)(3,4-dihydro-2H-pyrido[1,2-a]pyrimidin-3-yl)-methanon
sowie jeweils deren Hydroperchloratsalze ausgenommen sind.

2. Verbindungen gemäß Anspruch 1, **dadurch gekennzeichnet, daß** $R^1$ und $R^2$, unabhängig voneinander, jeweils für H, F, Cl, Br oder einen linearen oder verzweigten, ggf. wenigstens einfach substituierten $C_{1-6}$-Alkyl-Rest, bevorzugt für H oder einen Methyl-Rest, stehen.

3. Verbindungen gemäß Anspruch 1 oder 2, **dadurch gekennzeichnet, daß** $R^3$ für einen ggf. wenigstens einfach substituierten, 5-oder 6-gliedrigen, monocyclischen Aryl- oder Heteroaryl-Rest steht, der mit einem ggf. wenigstens einfach substituierten, ggf. wenigstens ein Heteroatom als Ringglied aufweisenden, mono-, bi- oder tricyclischen Ringsystem kondensiert sein kann, wobei die Ringe des Ringsystems jeweils 5 bis 7-gliedrig sind, vorzugsweise für einen wenigstens einfach substituierten Phenyl-Rest oder einen ggf. wenigstens einfach substituierten Phenyl-, Naphthyl-, Furanyl-, Thiophenyl-, Pyrrolyl-, Imidazolyl-, Pyrazolyl-, Pyridinyl-, Pyrimidinyl-, Chinolinyl- oder Isochinolinyl-Rest steht.

4. Verbindungen gemäß einem der Ansprüche 1 - 3, **dadurch gekennzeichnet, daß** sie ausgewählt ist aus der Gruppe bestehend aus:

   3-(2'-Naphthoyl)-3,4-dihydro-2*H* 8-methylpyrido[1,2-a]pyrimidin,

3-(2'-Naphthoyl)-3,4-dihydro-2*H*-6,8-dimethylpyrido[1,2-a]pyrimidin,
3-(4'-Fluorbenzoyl)-3,4-dihydro-2*H*-8-methylpyrido[1,2-a]pyrimidin,
3-Benzoyl-3,4-dihydro-2*H*-6,8-dimethylpyrido[1,2-a]pyrimidin,
3-(4'-Biphenylcarbonyl)-3,4-dihydro-2*H*-8-methylpyrido[1,2-a]pyrimidin,
3-(4'-Biphenylcarbonyl)-3,4-dihydro-2*H*-6-methylpyrido[1,2-a]pyrimidin,
(4-Ethoxy-phenyl)-(8-methyl-3,4-dihydro-2*H*-pyrido[1,2-a]pyrimidin-3-yl)-methanon,
Biphenyl-4-yl-(6,8-dimethyl-3,4-dihydro-2H-pyrido[1,2-a]pyrimidin-3-yl)-methanon,
(8-Methyl-3,4-dihydro-2H-pyrido[1,2-a]pyrimidin-3-yl)-(6-methyl-naphthalin-2-yl)-methanon, und
(6-Methoxy-naphthalin-2-yl)-(8-methyl-3,4-dihydro-2H-pyrido[1,2-a]pyrimidin-3-yl)-methanon,

ggf. in Form ihrer freien Base oder in Form ihres physiologisch verträglichen Salzes, insbesondere Hydroperchloratsalzes, oder jeweils in Form ihres Solvates, insbesondere Hydrates.

**5.** Verfahren zur Herstellung von substituierten Pyrido[1,2-a]pyrimidin-Verbindungen gemäß einem der Ansprüche 1 bis 4, **dadurch gekennzeichnet, daß** wenigstens eine Verbindung der allgemeinen Formel III,

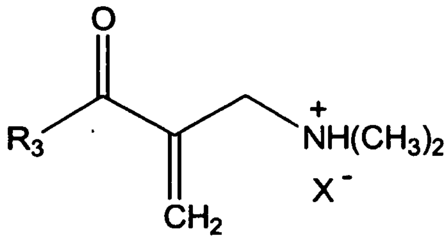

III,

worin $R^3$ die Bedeutung gemäß Anspruch 1 hat und $X^-$ für ein Chlorid- oder Bromid-Anion steht, mit wenigstens einer Verbindung der allgemeinen Formel IV,

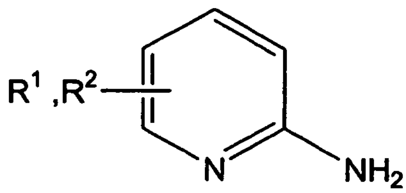

IV,

worin $R^1$ und $R^2$ die Bedeutung gemäß Anspruch 1 haben, in einem geeigneten Reaktionsmedium, vorzugsweise in einem Wasser/Alkohol-Gemisch, besonders bevorzugt in einem Wasser/Ethanol-Gemisch, unter Erhitzen, vorzungsweise unter Rückfluß, umgesetzt, und die so erhaltene Verbindung der allgemeinen Formel ggf. gereinigt und ggf. isoliert wird.

**6.** Arzneimittel enthaltend wenigstens eine substituierte Pyrido[1,2-a]pyrimidin-Verbindung der allgemeinen Formel II

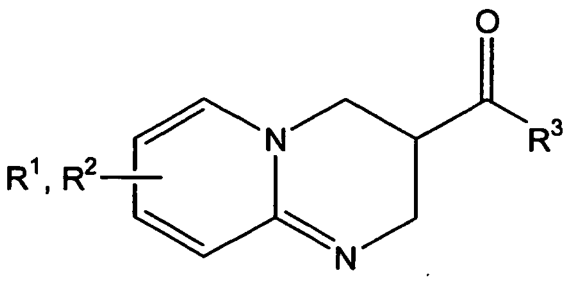

II,

worin
$R^1$ und $R^2$, unabhängig voneinander, jeweils für Wasserstoff, Halogen oder einen linearen oder verzweigten, ggf. wenigstens einfach substituierten Alkyl-Rest stehen und
$R^3$ für einen ggf. wenigstens einfach substituierten, monocyclischen Aryl- oder Heteroaryl-Rest steht, der mit einem ggf. wenigstens einfach substituierten, ggf. wenigstens ein Heteroatom als Ringglied aufweisenden, monocyclischen oder polycyklischen Ringsystem kondensiert sein kann,
ggf. in Form eines ihrer reinen Stereoisomeren, insbesondere Enantiomeren oder Diastereomeren, ihres Racemates oder in Form einer Mischung von Stereoisomeren, insbesondere der Enantiomeren oder Diastereomeren, in einem beliebigen Mischungsverhältnis, oder jeweils in Form ihrer freien Base oder in Form ihres physiologisch verträglichen Salzes, insbesondere Hydroperchloratsalzes, oder jeweils in Form ihres Solvates, insbesondere Hydrates.

**7.** Arzneimittel gemäß Anspruch 6, **dadurch gekennzeichnet, daß** die Reste $R^1$ und $R^2$, unabhängig voneinander, jeweils für H, F, Cl, Br oder für einen linearen oder verzweigten, ggf. wenigstens einfach substituierten $C_{1-6}$-Alkyl-Rest, bevorzugt für H oder einen Methyl-Rest stehen.

**8.** Arzneimittel gemäß Anspruch 6 oder 7, **dadurch gekennzeichnet, daß** der Rest $R^3$ für einen ggf. wenigstens einfach substituierten, 5-oder 6-gliedrigen, monocyclischen Aryl- oder Heteroaryl-Rest steht, der mit einem ggf. wenigstens einfach substituierten, ggf. wenigstens ein Heteroatom als Ringglied aufweisenden, mono-, bi- oder tricyclischen Ringsystem kondensiert sein kann, wobei die Ringe des Ringsystems jeweils 5 bis 7-gliedrig sind, vorzugsweise für einen ggf. wenigstens einfach substituierten Phenyl-, Naphthyl-, Furanyl-, Thiophenyl-, Pyrrolyl-, Imidazolyl-, Pyrazolyl-, Pyridinyl-, Pyrimidinyl-, Chinolinyl-oder Isochinolinyl-Rest, besonders bevorzugt für einen ggf. wenigstens einfach substituierten Phenyl-, oder Naphtylrest steht.

**9.** Arzneimittel gemäß einem der Ansprüche 6 - 8, **dadurch gekennzeichnet, daß** die Verbindung der allgemeinen Formel II ausgewählt ist aus der Gruppe bestehend aus:

3-(2'-Naphthoyl)-3,4-dihydro-2*H*-8-methylpyrido[1,2-a]pyrimidin,
3-(2'-Naphthoyl)-3,4-dihydro-2*H*-6,8-dimethylpyrido[1,2-a]pyrimidin,
3-(4'-Fluorbenzoyl)-3,4-dihydro-2*H*-8-methylpyrido[1,2-a]pyrimidin,
3-Benzoyl-3,4-dihydro-2*H*-6,8-dimethylpyrido[1 ,2-a]pyrimidin,
3-(4'-Biphenylcarbonyl)-3,4-dihydro-2*H*-8-methylpyrido[1,2-a]pyrimidin,
3-(4'-Biphenylcarbonyl)-3,4-dihydro-2*H*-6-methylpyrido[1,2-a]pyrimidin,
3-(4'-Brombenzoyl)-3,4-dihydro-2*H*-8-methylpyrido[1,2-a]pyrimidin,
3-(4'-Chlorbenzoyl)-3,4-dihydro-2*H*-8-methylpyrido[1,2-a]pyrimidin,
3-Benzoyl-3,4-dihydro-2*H*-8-methylpyrido[1,2-a]pyrimidin,
(4-Ethoxy-phenyl)-(8-methyl-3,4-dihydro-2H-pyrido[1,2-a]pyrimidin-3-yl)-methanon,
Biphenyl-4-yl-(6,8-dimethyl-3,4-dihydro-2H-pyrido[1,2-a]pyrimidin-3-yl)-methanon,
(8-Methyl-3,4-dihydro-2H-pyrido[1,2-a]pyrimidin-3-yl)-(6-methyl-naphthalin-2-yl)-methanon, und
(6-Methoxy-naphthalin-2-yl)-(8-methyl-3,4-dihydro-2H-pyrido[1,2-a]pyrimidin-3-yl)-methanon,

ggf. in Form ihrer freien Base oder in Form ihres physiologisch verträglichen Salzes, insbesondere Hydroperchloratsalzes, oder jeweils in Form ihres Solvates, insbesondere Hydrates.

**10.** Arzneimittel gemäß einem der Ansprüche 6 - 9 zur Inhibierung der Stickstoffmonoxid-Synthase (NOS), zur Behandlung und/oder Prophylaxe von Migräne, zur Bekämpfung von Schmerz, vorzugsweise chronischem Schmerz und/oder Entzündungsschmerz, zur Behandlung von septischem Schock, neurodegenerativen Erkrankungen, vorzugsweise multipler Sklerose, Morbus Parkinson, Morbus Alzheimer und/oder Morbus Huntington, Entzündungen, Entzündungsschmerz, cerebraler Ischämie, Diabetes, Meningitis, Arteriosklerose, zur Wundheilung, zur Behandlung von Tumoren, zur Inhibierung der Angiogenese, als Antibiotikum, vorzugsweise zur Auslösung einer unspezifischen Immunantwort gegen Mikroorganismen.

**11.** Verwendung wenigstens einer Verbindung gemäß einem der Ansprüche 6 - 9 zur Herstellung eines Arzneimittels zur Inhibierung der Stickstoffmonoxid-Synthase (NOS).

**12.** Verwendung wenigstens einer Verbindung gemäß einem der Ansprüche 6 - 9 zur Herstellung eines Arzneimittels zur Bekämpfung von Schmerz, vorzugsweise chronischem Schmerz und/oder Entzündungsschmerz.

**13.** Verwendung wenigstens einer Verbindung gemäß einem der Ansprüche 6 - 9 zur Herstellung eines Arzneimittels zur Prophylaxe und/oder Behandlung von Migräne.

**14.** Verwendung wenigstens einer Verbindung gemäß einem der Ansprüche 6-9 zur Herstellung eines Arzneimittels zur Behandlung von septischem Schock.

**15.** Verwendung wenigstens einer Verbindung gemäß einem der Ansprüche 6 - 9 zur Herstellung eines Arzneimittels zur Behandlung von neurodegenerativen Erkrankungen, vorzugsweise multipler Sklerose, Morbus Parkinson, Morbus Alzheimer, und/ oder Morbus Huntington.

**16.** Verwendung wenigstens einer Verbindung gemäß einem der Ansprüche 6 - 9 zur Herstellung eines Arzneimittels zur Behandlung von Entzündungen.

**17.** Verwendung wenigstens einer Verbindung gemäß einem der Ansprüche 6 - 9 zur Herstellung eines Arzneimittels zur Behandlung von cerebraler Ischämie.

**18.** Verwendung wenigstens einer Verbindung gemäß einem der Ansprüche 6 - 9 zur Herstellung eines Arzneimittels zur Behandlung von Diabetes.

**19.** Verwendung wenigstens einer Verbindung gemäß einem der Ansprüche 6 - 9 zur Herstellung eines Arzneimittels zur Behandlung von Meningitis.

**20.** Verwendung wenigstens einer Verbindung gemäß einem der Ansprüche 6 - 9 zur Herstellung eines Arzneimittels zur Behandlung von Arteriosklerose.

**21.** Verwendung wenigstens einer Verbindung gemäß einem der Ansprüche 6 - 9 zur Herstellung eines Arzneimittels zur Wundheilung.

**22.** Verwendung wenigstens einer Verbidung gemäß einem der Ansprüche 6 - 9 zur Herstellung eines Arzneimittels zur Behandlung von Tumoren.

**23.** Verwendung wenigstens einer Verbindung gemäß einem der Ansprüche 6 - 9 zur Herstellung eines Arzneimittels zur Inhibierung der Angiogenese.

**24.** Verwendung wenigstens einer Verbindung gemäß einem der Ansprüche 6 - 9 zur Herstellung eines Arzneimittels als Antibiotikum, vorzugsweise zur Auslösung einer unspezifischen Immunantwort gegen Mikroorganismen.

## Claims

**1.** Substituted pyrido[1,2-a]pyrimidine compounds of the general formula I

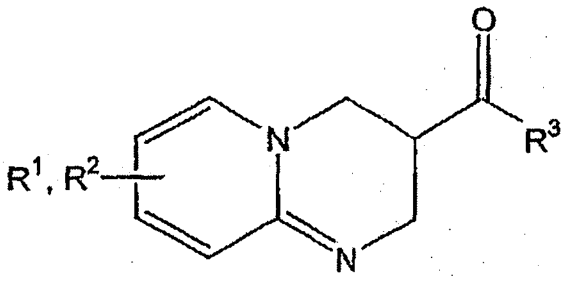

I

in which
$R^1$ and $R^2$, mutually independently, in each case denote hydrogen, halogen or a linear or branched, optionally at least monosubstituted alkyl residue, and
$R^3$ denotes an optionally at least monosubstituted, monocyclic aryl or heteroaryl residue, which may be fused with an optionally at least monosubstituted monocyclic or polycyclic ring system optionally comprising at least one heteroatom as a ring member,
optionally in the form of one of the pure stereoisomers thereof, in particular enantiomers or diastereomers, the racemate thereof or in the form of a mixture of stereoisomers, in particular the enantiomers or diastereomers, in any desired mixing ratio, or in each case in the form of the free base thereof or in the form of the physiologically acceptable salt thereof, in particular hydroperchlorate salt, or in each case in the form of the solvate thereof, in particular hydrate, wherein compounds of the general formula I, in which $R^1$ and $R^2$ denote hydrogen and a methyl residue or $R^1$ and $R^2$ in each case denote hydrogen and $R^3$ in each case denotes a phenyl residue which is unsubstituted or monosubstituted with a methyl residue, methoxy residue, Cl or Br together with the compounds
(3,4-dihydro-9-methyl-2*H*-pyrido[1,2-a]pyrimidin-3-yl)(3,4-dimethoxyphenyl)-methanone,
(3,4-dihydro-6-methyl-2*H*-pyrido[1,2-a]pyrimidin-3-yl)(3,4-dimethoxyphenyl)-methanone,
(3,4-dihydro-2*H*-pyrido[1,2-a]pyrimidin-3-yl) (3,4-dimethoxyphenyl)-methanone and
(3,4-dichlorophenyl)(3,4-dihydro-2*H*-pyrido[1,2-a]pyrimidin-3-yl)-methanone
and in each case the hydroperchlorate salts thereof are excepted.

2. Compounds according to claim 1, **characterised in that** $R^1$ and $R^2$, mutually independently, in each case denote H, F, Cl, Br or a linear or branched, optionally at least monosubstituted $C_{1-5}$ alkyl residue, preferably H or a methyl residue.

3. Compounds according to claim 1 or claim 2, **characterised in that** $R^3$ denotes an optionally at least monosubstituted, 5- or 6-membered, monocyclic aryl or heteroaryl residue, which may be fused with an optionally at least monosubstituted mono-, di- or tricyclic ring system optionally comprising at least one heteroatom as a ring member, wherein the rings of the ring system are in each case 5- to 7-membered, preferably an at least monosubstituted phenyl residue or an optionally at least monosubstituted phenyl, naphthyl, furanyl, thiophenyl, pyrrolyl, imidazolyl, pyrazolyl, pyridinyl, pyrimidinyl, quinolinyl or isoquinolinyl residue.

4. Compounds according to any one of claims 1-3, **characterised in that** they are selected from the group consisting of:

3-(2'-naphthoyl)-3,4-dihydro-2*H*-8-methylpyrido[1,2-a]pyrimidine,
3-(2'-naphthoyl)-3,4-dihydro-2*H*-6,8-dimethylpyrido[1,2-a]pyrimidine,
3-(4'-fluorobenzoyl)-3,4-dihydro-2*H*-8-methylpyrido[1,2-a]pyrimidine,
3-benzoyl-3,4-dihydro-2*H*-6,8-dimethylpyrido [1,2-a] pyrimidine,
3-(4'-biphenylcarbonyl)-3,4-dihydro-2*H*-8-methylpyrido(1,2-a)pyrimidine,
3-(4'-biphenylcarbonyl)-3,4-dihydro-2*H*-6-methylpyrido[1,2-a]pyrimidine,
(4-ethoxy-phenyl)-(8-methyl-3,4-dihydro-2*H*-pyrido[1,2-a]pyrimidin-3-yl)-methanone,
biphenyl-4-yl-(6,8-dimethyl-3,4-dihydro-2*H*-pyrido[1,2-a]pyrimidin-3-yl)-methanone,
(8-methyl-3,4-dihydro-2*H*-pyrido[1,2-a]pyrimidin-3-yl)-(6-methyl-naphthalen-2-yl)-methanone, and
(6-methoxy-naphthalen-2-yl)-(8-methyl-3,4-dihydro-2*H*-pyrido[1,2-a]pyrimidin-3-yl)-methanone,

optionally in the form of the free base thereof or in the form of the physiologically acceptable salt thereof, in particular hydroperchlorate salt, or in each case in the form of the solvate thereof, in particular hydrate.

**5.** A process for the production of substituted pyrido[1,2-a]pyrimidine compounds according to any one of claims 1 to 4, **characterised in that** at least one compound of the general formula III,

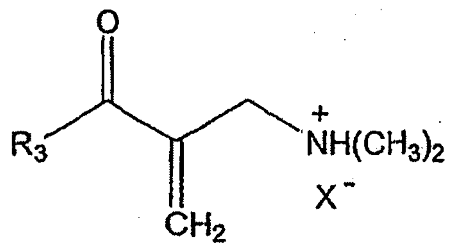

III,

in which $R^3$ has the meaning according to claim 1 and $X^-$ denotes a chloride or bromide anion, is reacted with at least one compound of the general formula IV,

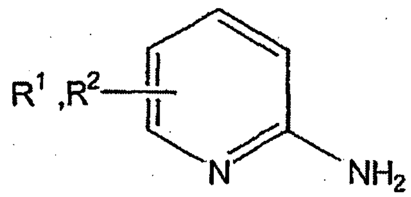

IV,

in which $R^1$ and $R^2$ have the meaning according to claim 1, in a suitable reaction medium, preferably in a water/alcohol mixture, particularly preferably in a water/ethanol mixture, with heating, preferably with refluxing, and the resultant compound of the general formula I is optionally purified and optionally isolated.

**6.** A pharmaceutical preparation containing at least one substituted pyrido[1,2-a]pyrimidine compound of the general formula II

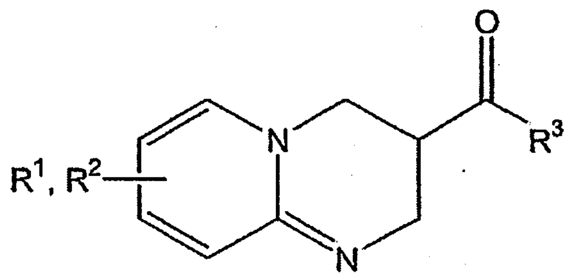

II,

in which
$R^1$ and $R^2$, mutually independently, in each case denote hydrogen, halogen or a linear or branched, optionally at least monosubstituted alkyl residue, and
$R^3$ denotes an optionally at least monosubstituted, monocyclic aryl or heteroaryl residue, which may be fused with an optionally at least monosubstituted monocyclic or polycyclic ring system optionally comprising at least one heteroatom as a ring member,
optionally in the form of one of the pure stereoisomers thereof, in particular enantiomers or diastereomers, the

racemate thereof or in the form of a mixture of stereoisomers, in particular the enantiomers or diastereomers, in any desired mixing ratio, or in each case in the form of the free base thereof or in the form of the physiologically acceptable salt thereof, in particular hydroperchlorate salt, or in each case in the form of the solvate thereof, in particular hydrate.

**7.** A pharmaceutical preparation according to claim 6, **characterised in that** the residues $R^1$ and $R^2$, mutually independently, in each case denote H, F, Cl, Br or a linear or branched, optionally at least monosubstituted $C_{1-6}$ alkyl residue, preferably H or a methyl residue.

**8.** A pharmaceutical preparation according to claim 6 or claim 7, **characterised in that** the residue $R^3$ denotes an optionally at least monosubstituted 5- or 6-membered, monocyclic aryl or heteroaryl residue, which may be fused with an optionally at least monosubstituted, mono-, di- or tricyclic ring system optionally comprising at least one heteroatom as a ring member, wherein the rings of the ring system are in each case 5- to 7-membered, preferably an optionally at least monosubstituted phenyl, naphthyl, furanyl, thiophenyl, pyrrolyl, imidazolyl, pyrazolyl, pyridinyl, pyrimidinyl, quinolinyl or isoquinolinyl residue, particularly preferably an optionally at least monosubstituted phenyl or naphthyl residue.

**9.** A pharmaceutical preparation according to any one of claims 6-8, **characterised in that** the compound of the general formula II is selected from the group consisting of:

3-(2'-naphthoyl)-3,4-dihydro-2*H*-8-methylpyrido[1,2-a]pyrimidine,
3-(2'-naphthoyl)-3,4-dihydro-2*H*-6,8-dimethylpyrido[1,2-a]pyrimidine,
3-(4'-fluorobenzoyl)-3,4-dihydro-2*H*-8-methylpyrido[1,2-a]pyrimidine,
3-benzoyl-3,4-dihydro-2*H*-6,8-dimethylpyrido[1,2-a]pyrimidine,
3-(4'-biphenylcarbonyl)-3,4-dihydro-2*H*-8-methylpyrido[1,2-a]pyrimidine,
3-(4'-biphenylcarbonyl)-3,4-dihydro-2*H*-6-methylpyrido[1,2-a]pyrimidine,
3-(4'-bromobenzoyl)-3,4-dihydro-2*H*-8-methylpyrido[1,2-a]pyrimidine,
3-(4'-chlorobenzoyl)-3,4-dihydro-2*H*-8-methylpyrido[1,2-a]pyrimidine,
3-benzoyl-3,4-dihydro-2*H*-8-methylpyrido[1,2-a]pyrimidine,
(4-ethoxy-phenyl)-(8-methyl-3,4-dihydro-2*H*-pyrido[1,2-a]pyrimidin-3-yl)-methanone,
biphenyl-4-yl-(6,8-dimethyl-3,4-dihydro-2*H*-pyrido[1,2-a]pyrimidin-3-yl)-methanone,
(8-methyl-3,4-dihydro-2*H*-pyrido[1,2-a]pyrimidin-3-yl)-(6-methyl-naphthalen-2-yl)-methanone, and
(6-methoxy-naphthalen-2-yl)-(8-methyl-3,4-dihydro-2*H*-pyrido[1,2-a]pyrimidin-3-yl)-methanone,

optionally in the form of the free base thereof or in the form of the physiologically acceptable salt thereof, in particular hydroperchlorate salt, or in each case in the form of the solvate thereof, in particular hydrate.

**10.** A pharmaceutical preparation according to any one of claims 6-9 for inhibiting nitrogen monoxide synthase (NOS), for the treatment and/or prevention of migraine, for combatting pain, preferably chronic pain and/or inflammatory pain, for the treatment of septic shock, neurodegenerative diseases, preferably multiple sclerosis, Parkinson's disease, Alzheimer's disease and/or Huntington's chorea, inflammation, inflammatory pain, cerebral ischaemia, diabetes, meningitis, arteriosclerosis, for wound healing, for the treatment of tumours, for inhibiting angiogenesis, as an antibiotic, preferably for triggering a nonspecific immune response against microorganisms.

**11.** Use of at least one compound according to any one of claims 6-9 for the production of a pharmaceutical preparation for inhibiting nitrogen monoxide synthase (NOS).

**12.** Use of at least one compound according to any one of claims 6-9 for combatting pain, preferably chronic pain and/or inflammatory pain.

**13.** Use of at least one compound according to any one of claims 6-9 for the production of a pharmaceutical preparation for the prevention and/or treatment of migraine.

**14.** Use of at least one compound according to any one of claims 6-9 for the production of a pharmaceutical preparation for the treatment of septic shock.

**15.** Use of at least one compound according to any one of claims 6-9 for the production of a pharmaceutical preparation for the treatment of neurodegenerative diseases, preferably multiple sclerosis, Parkinson's disease, Alzheimer's disease, and/or Huntington's chorea.

16. Use of at least one compound according to any one of claims 6-9 for the production of a pharmaceutical preparation for the treatment of inflammation.

17. Use of at least one compound according to any one of claims 6-9 for the production of a pharmaceutical preparation for the treatment of cerebral ischaemia.

18. Use of at least one compound according to any one of claims 6-9 for the production of a pharmaceutical preparation for the treatment of diabetes.

19. Use of at least one compound according to any one of claims 6-9 for the production of a pharmaceutical preparation for the treatment of meningitis.

20. Use of at least one compound according to any one of claims 6-9 for the production of a pharmaceutical preparation for the treatment of arteriosclerosis.

21. Use of at least one compound according to any one of claims 6-9 for the production of a pharmaceutical preparation for wound healing.

22. Use of at least one compound according to any one of claims 6-9 for the production of a pharmaceutical preparation for the treatment of tumours.

23. Use of at least one compound according to any one of claims 6-9 for the production of a pharmaceutical preparation for inhibiting angiogenesis.

24. Use of at least one compound according to any one of claims 6-9 for the production of a pharmaceutical preparation as an antibiotic, preferably for triggering a nonspecific immune response against microorganisms.

## Revendications

1. Pyrido[1,2-a]pyrimidines substituées de Formule générale I

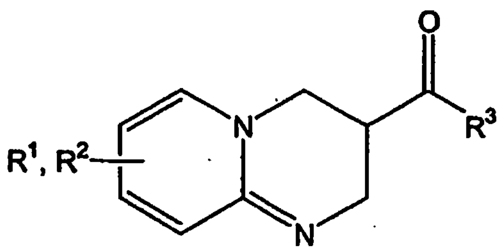

I

dans laquelle
$R^1$ et $R^2$ représentent chacun indépendamment de l'autre un atome d'hydrogène, un atome d'halogène ou un radical alkyle à chaîne droite ou ramifiée, éventuellement au moins une fois substitué, et
$R^3$ représente un radical aryle ou hétéroaryle monocyclique, éventuellement au moins une fois monosubstitué, qui peut être condensé avec un système cyclique, monocyclique ou polycyclique, éventuellement au moins une fois substitué, comportant éventuellement au moins un hétéroatome en tant que chaînon,
éventuellement sous forme de l'un de leurs stéréoisomères purs, en particulier de leurs énantiomères ou de leurs diastéréoisomères, de leur racémate, ou sous forme d'un mélange de stéréoisomères, en particulier des énantiomères ou des diastéréoisomères, selon un rapport de mélange quelconque, ou dans tous les cas sous la forme de leur base libre, ou sous la forme de leur sel acceptable d'un point de vue physiologique, en particulier de leur sel hydroperchlorate, ou dans tous les cas sous forme de leur produit de solvatation, en particulier de leur hydrate,
à l'exception des composés de formule générale I dans laquelle $R^1$ et $R^2$ représentent un atome d'hydrogène et un groupe méthyle, ou encore $R^1$ et $R^2$ représentent chacun un atome d'hydrogène, et $R^3$ représente dans chaque cas un groupe phényle non substitué, ou une fois substitué par un substituant méthyle, un substituant méthoxy, Cl

ou Br, ainsi que des composés suivants :

(3,4-dihydro-9-méthyl-2H-pyrido[1,2-a]pyrimidine-3-yl)(3,4-diméthoxyphényl)-méthanone,
(3,4-dihydro-6-méthyl-2H-pyrido[1,2-a]pyrimidine-3-yl)(3,4-diméthoxyphényl)-méthanone,
(3,4-dihydro-2H-pyrido[1,2-a]pyrimidine-3-yl)(3,4-diméthoxyphényl)-méthanone, et
(3,4-dichlorophényl)(3,4-dihydro-2H-pyrido[1,2-a]pyrimidine-3-yl)-méthanone

ainsi que de leurs sels hydroperchlorates.

**2.** Composés selon la revendication 1, **caractérisés en ce que** $R^1$ et $R^2$ représentent chacun indépendamment de l'autre H, F, C1 ou Br, ou un radical alkyle en $C_{1-6}$ à chaîne droite ou ramifiée, éventuellement au moins une fois substitué, de préférence H ou un radical méthyle.

**3.** Composés selon la revendication 1 ou 2, **caractérisés en ce que** $R^3$ représente un radical aryle ou hétéroaryle monocyclique à 5 ou 6 chaînons, éventuellement au moins une fois substitué, qui peut être condensé avec un système cyclique monocyclique, bicyclique ou tricyclique, éventuellement au moins une fois substitué, comportant éventuellement au moins un hétéroatome en tant que chaînon, les noyaux du système cyclique ayant chacun 5 à 7 chaînons, de préférence un radical phényle au moins une fois substitué, ou un radical phényle, naphtyle, furannyle, thiophényle, pyrrolyle, imidazolyle, pyrazolyle, pyridinyle, pyrimidinyle, quinoléinyle ou isoquinoléinyle, éventuellement au moins une fois substitué.

**4.** Composés selon l'une des revendications 1 à 3, **caractérisés en ce qu'**ils sont choisis dans l'ensemble consistant en les composés suivants :

3-(2'-naphtoyl)-3,4-dihydro-2*H*-8-méthylpyrido[1,2-a]pyrimidine,
3-(2'-naphtoyl)-3,4-dihydro-2*H*-6,8-méthylpyrido[1,2-a]pyrimidine,
3-(4'-fluorobenzoyl)-3,4-dihydro-2*H*-8-méthylpyrido[1,2-a]pyrimidine,
3-benzoyl-3,4-dihydro-2*H*-6,8-méthylpyrido[1,2-a]-pyrimidine,
3-(4'-biphénylcarbonyl)-3,4-dihydro-2*H*-8-méthyl-pyrido[1,2-a]pyrimidine,
3-(4'-biphénylcarbonyl)-3,4-dihydro-2*H*-6-méthyl-pyrido[1,2-a]pyrimidine,
(4-éthoxyphényl)-(8-méthyl-3,4-dihydro-2*H*-pyrido[1,2-a]pyrimidine-3-yl)-méthanone,
Biphényle-4-yl-(6,8-méthyl-3,4-dihydro-2*H*-pyrido[1,2-a]pyrimidine-3-yl)-méthanone,
(8-méthyl-3,4-dihydro-2*H*-pyrido[1,2-a]pyrimidine-3-yl)-(6-méthylnaphtalène-2-yl)-méthanone, et
(6-méthoxynaphtalène-2-yl)-(8-méthyl-3,4-dihydro-2*H*-pyrido[1,2-a]pyrimidine-3-yl)-méthanone,

éventuellement sous forme de leur base libre, ou sous forme de leur sel acceptable d'un point de vue physiologique, en particulier de leur sel hydroperchlorate, ou dans tous les cas sous forme de leur produit de solvatation, en particulier de leur hydrate.

**5.** Procédé de préparation de pyrido[1,2-a]-pyrimidines substituées selon l'une des revendications 1 à 4, **caractérisé en ce qu'**on fait réagir au moins un composé de formule générale III

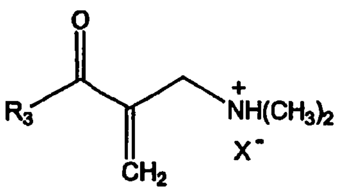

III,

dans laquelle $R^3$ a les significations selon la revendication 1 et $X^-$ est un anion chlorure ou bromure, avec au moins un composé de formule générale IV

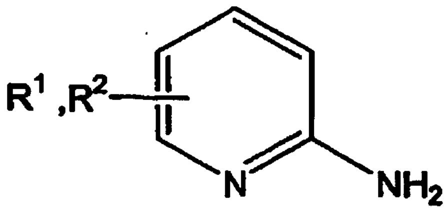

IV,

dans laquelle $R^1$ et $R^2$ ont les significations données dans la revendication 1, dans un milieu réactionnel approprié, de préférence dans un mélange d'eau et d'alcool, d'une manière particulièrement préférée dans un mélange d'eau et d'éthanol, en présence d'un chauffage, de préférence au reflux, et **en ce qu'**on va éventuellement purifier et éventuellement isoler le composé ainsi obtenu de formule générale I.

**6.** Médicament contenant au moins une pyrido[1,2-a]pyrimidine substituée de formule générale II

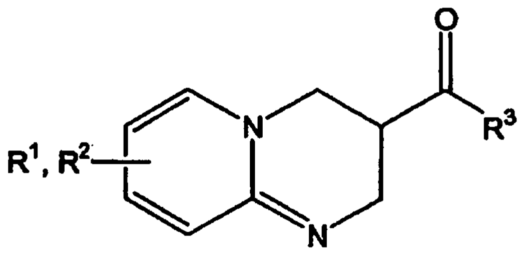

II

dans laquelle
$R^1$ et $R^2$ représentent chacun indépendamment de l'autre un atome d'hydrogène, un atome d'halogène ou un radical alkyle à chaîne droite ou ramifiée, éventuellement au moins une fois substitué, et
$R^3$ représente un radical aryle ou hétéroaryle monocyclique, éventuellement au moins une fois monosubstitué, qui peut être condensé avec un système cyclique, monocyclique ou polycyclique, éventuellement au moins une fois substitué, comportant éventuellement au moins un hétéroatome en tant que chaînon,
éventuellement sous forme de l'un de leurs stéréoisomères purs, en particulier de leurs énantiomères ou de leurs diastéréoisomères, de leur racémate, ou sous forme d'un mélange de stéréoisomères, en particulier des énantiomères ou des diastéréoisomères, selon un rapport de mélange quelconque, ou dans tous les cas sous la forme de leur base libre, ou sous la forme de leur sel acceptable d'un point de vue physiologique, en particulier de leur sel hydroperchlorate, ou dans tous les cas sous forme de leur produit de solvatation, en particulier de leur hydrate.

**7.** Médicament selon la revendication 6, **caractérisé en ce que** les radicaux $R^1$ et $R^2$ représentent chacun indépendamment de l'autre H, F, Cl, Br, ou un radical alkyle en $C_{1-6}$ à chaîne droite ou ramifiée, éventuellement au moins une fois substitué, de préférence H ou un radical méthyle.

**8.** Médicament selon la revendication 6 ou 7, **caractérisé en ce que** le radical $R^3$ représente un radical aryle ou hétéroaryle monocyclique à 5 ou 6 chaînons, éventuellement au moins une fois substitué, qui peut être condensé avec un système cyclique monocyclique, bicyclique ou tricyclique, éventuellement au moins une fois substitué, comportant éventuellement au moins un hétéroatome en tant que chaînon, les noyaux du système cyclique ayant chacun 5 à 7 chaînons, de préférence un radical phényle au moins une fois substitué, ou un radical phényle, naphtyle, furannyle, thiophényle, pyrrolyle, imidazolyle, pyrazolyle, pyridinyle, pyrimidinyle, quinoléinyle, d'une manière particulièrement préférée un radical phényle ou naphtyle éventuellement au moins une fois substitué.

**9.** Médicament selon l'une des revendications 6 à 8, **caractérisé en ce que** le composé de formule générale II est choisi dans l'ensemble consistant en les composés suivants:

3-(2'-naphtoyl)-3,4-dihydro-2*H*-8-méthylpyrido[1,2-a]pyrimidine,
3-(2'-naphtoyl)-3,4-dihydro-2*H*-6,8-diméthylpyrido[1,2-a]pyrimidine,
3-(4'-fluorobenzoyl)-3,4-dihydro-2*H*-8-méthylpyrido[1,2-a]pyrimidine,
3-benzoyl-3,4-dihydro-2*H*-6,8-diméthylpyrido[1,2-a]pyrimidine,
3-(4'-biphénylcarbonyl)-3,4-dihydro-2*H*-8-méthyl-pyrido[1,2-a]pyrimidine,
3-(4'-biphénylcarbonyl)-3,4-dihydro-2*H*-6-méthyl-pyrido[1,2-a]pyrimidine,
3-(4'-bromobenzoyl)-3,4-dihydro-2*H*-8-méthylpyrido[1,2-a]pyrimidine,
3-(4'-chlorobenzoyl)-3,4-dihydro-2*H*-8-méthylpyrido[1,2-a]pyrimidine,
3-benzoyl-3,4-dihydro-2*H*-8-méthylpyrido[1,2-a]-pyrimidine,
(4-éthoxyphényl)-(8-méthyl-3,4-dihydro-2*H*-pyrido[1,2-a]pyrimidine-3-yl)-méthanone,
Biphényle-4-yl)-(6,8-méthyl-3,4-dihydro-2*H*-pyrido[1,2-a]pyrimidine-3-yl)-méthanone,
(8-méthyl-3,4-dihydro-2*H*-pyrido[1,2-a]pyrimidine-3-yl)-(6-méthylnaphtalène-2-yl)-méthanone, et
(6-méthoxynaphtalène-2-yl)-(8-méthyl-3,4-dihydro-2*H*-pyrido[1,2-a]pyrimidine-3-yl)-méthanone,

éventuellement sous forme de leur base libre, ou sous forme de leur sel acceptable d'un point de vue physiologique, en particulier de leur sel hydroperchlorate, ou dans tous les cas sous forme de leur produit de solvatation, en particulier de leur hydrate.

**10.** Médicament selon l'une des revendications 6 à 9, pour inhiber la (monoxyde d'azote)-synthase (NOS), pour le traitement et/ou la prophylaxie de la migraine, pour maîtriser la douleur, de préférence les douleurs chroniques et/ou les douleurs inflammatoires, pour le traitement du choc septique, des maladies neurodégénératives, de préférence la sclérose en plaques, la maladie de Parkinson, la maladie d'Alzheimer et/ou la maladie de Huntington, des inflammations, des douleurs inflammatoires, de l'ischémie cérébrale, du diabète, de la méningite, de l'artériosclérose, pour la cicatrisation, pour le traitement des tumeurs, pour l'inhibition de l'angiogenèse, en tant qu'antibiotiques, de préférence pour déclencher une réponse immune non spécifique contre des microorganismes.

**11.** Utilisation d'au moins un composé selon l'une des revendications 6 à 9 pour préparer un médicament destiné à inhiber la (monoxyde d'azote)-synthase (NOS).

**12.** Utilisation d'au moins un composé selon l'une des revendications 6 à 9 pour préparer un médicament destiné à la maîtrise de la douleur, de préférence des douleurs chroniques et/ou des maladies inflammatoires.

**13.** Utilisation d'au moins un composé selon l'une des revendications 6 à 9 pour préparer un médicament destiné à la prophylaxie et/ou du traitement de la migraine.

**14.** Utilisation d'au moins un composé selon l'une des revendications 6 à 9 pour préparer un médicament destiné au traitement du choc septique.

**15.** Utilisation d'au moins un composé selon l'une des revendications 6 à 9 pour préparer un médicament destiné au traitement des maladies neurodégénératives, de préférence la sclérose en plaques, la maladie de Parkinson, la maladie d'Alzheimer et/ou la maladie de Huntington.

**16.** Utilisation d'au moins un composé selon l'une des revendications 6 à 9 pour préparer un médicament destiné au traitement des inflammations.

**17.** Utilisation d'au moins un composé selon l'une des revendications 6 à 9 pour préparer un médicament destiné au traitement de l'ischémie cérébrale.

**18.** Utilisation d'au moins un composé selon l'une des revendications 6 à 9 pour préparer un médicament destiné au traitement du diabète.

**19.** Utilisation d'au moins un composé selon l'une des revendications 6 à 9 pour préparer un médicament destiné au traitement de la méningite.

**20.** Utilisation d'au moins un composé selon l'une des revendications 6 à 9 pour préparer un médicament destiné au traitement de l'artériosclérose.

**21.** Utilisation d'au moins un composé selon l'une des revendications 6 à 9 pour préparer un médicament destiné à la

cicatrisation.

**22.** Utilisation d'au moins un composé selon l'une des revendications 6 à 9 pour préparer un médicament destiné au traitement des tumeurs.

**23.** Utilisation d'au moins un composé selon l'une des revendications 6 à 9 pour préparer un médicament destiné à l'inhibition de l'angiogenèse.

**24.** Utilisation d'au moins un composé selon l'une des revendications 6 à 9 pour préparer un médicament servant d'antibiotique, de préférence pour déclencher une réponse immune non spécifique contre des microorganismes.